(19) Europäisches Patentamt
European Patent Office
Office européen des brevets

(11) Veröffentlichungsnummer : **0 565 484 A2**

(12) # EUROPÄISCHE PATENTANMELDUNG

(21) Anmeldenummer : **93810202.7**

(51) Int. Cl.[5] : **C07K 5/02,** A61K 37/64

(22) Anmeldetag : **22.03.93**

(30) Priorität : **01.04.92 CH 1052/92**

(43) Veröffentlichungstag der Anmeldung : **13.10.93 Patentblatt 93/41**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Bold, Guido, Dr.**
**Bleumatthöhe 16**
**CH-5264 Gipf-Oberfrick (CH)**
Erfinder : **Fässler, Alexander, Dr.**
**Sonnenblickstrasse 10**
**CH-3063 Ittigen (CH)**
Erfinder : **Lang, Marc, Dr.**
**Rue de Valdoie 24**
**F-68200 Mulhouse (FR)**
Erfinder : **Schneider, Peter, Dr.**
**Bäumliackerstrasse 8**
**CH-4103 Bottmingen (CH)**

(54) **Morpholin- und Thiomorpholin-4-ylamide mit HIV-Protease hemmender Wirkung.**

(57) Beschrieben sind Verbindungen der Formel I,

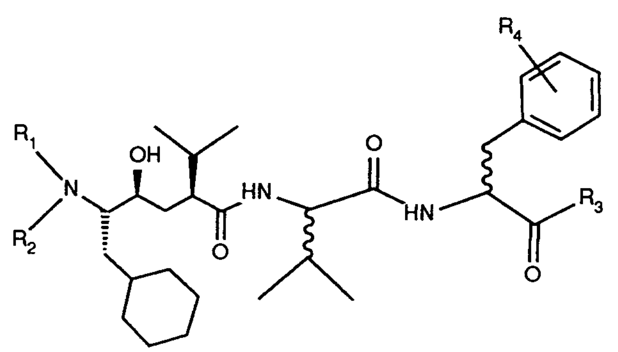

(I),

worin $R_1$ Wasserstoff oder Niederalkyl, $R_2$ Wasserstoff, Niederalkyl, Niederalkoxycarbonyl, Phenyl- oder Naphthyl-niederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu dem bindenden Stickstoffatom ein oder mehrere weitere Heteroatome ausgewählt aus unsubstituiertem oder durch einen $C_1$-$C_4$-Alkylrest substituiertem NH, O, S, S=O oder $SO_2$ als Ringglied enthält, Niederalkanoyl, Phenyl- oder Naphthylniederalkanoyl oder Niederalkansulfonyl bedeutet, $R_3$ Morpholino, Thiomorpholino, S-Oxothiomorpholino oder S,S-Dioxothiomorpholino bedeutet, wobei der Rest Heterocyclyl in Heterocyclylcarbonyl $R_2$ dieselbe Bedeutung wie $R_3$ oder eine von $R_3$ unterschiedliche Bedeutung hat, und $R_4$ Wasserstoff, Hydroxy, $C_1$-$C_4$-Alkoxy, Cyano, Trifluormethyl oder Fluor bedeuten, sowie Salze dieser Verbindungen, sofern salzbildende Gruppen vorliegen. Diese Verbindungen sind HIV-Proteasehemmer und werden verwendet zur Therapie von AIDS.

EP 0 565 484 A2

Die Erfindung betrifft nicht hydrolysierbare Analoga für durch Aspartatproteasen spaltbare Peptide, nämlich 5-Amino-4-hydroxy-hexanoyl-valyl-phenylalanylderivate, Verfahren zu ihrer Herstellung, pharmazeutische Präparate, die diese Peptidanaloga enthalten, und deren Verwendung als Arzneimittel oder zur Herstellung pharmazeutischer Präparate zur Bekämpfung retroviral bedingter Erkrankungen.

AIDS ist nach heutigem Wissen eine durch das zu den Lentiviren gehörende Retrovirus HIV ("Human Immunodeficiency Virus") hervorgerufene Erkrankung des Immunsystems. Diese Erkrankung betrifft nach Schätzungen der WHO rund 10 Millionen Menschen und breitet sich immer weiter aus. Die Erkrankung führt praktisch stets zum Tod des Patienten Eine effektive Behandlung, die zu einer Heilung führt, ist derzeit nicht möglich.

AIDS geht mit einer selektiven Verminderung von "T4-Helper/Inducer"-Lymphocyten einher, wobei erschwerend Komplikationen, die auf verminderter Leistungsfähigkeit des Immunsystems beruhen, wie opportunistische Infektionen, auftreten.

Bisher konnten die Retroviren HIV-1 und HIV-2 (HIV steht für "Human Immunodeficiency Virus") als ätiologisches Agens für die Erkrankung identifiziert und molekularbiologisch charakterisiert werden.

Therapeutische Ansätze zur Behandlung beruhen in erster Linie auf den molekularbiologischen Erkenntnissen. Insbesondere die Suche nach solchen Präparaten ist interessant, welche die Vermehrung des Virus selbst beeinträchtigen, ohne die intakten Zellen und Gewebe der Patienten zu schädigen. Bisher sind lediglich Hemmstoffe der Reversen Transcriptase, eines für HIV spezifischen Enzymes, das für die Weitergabe des Erbgutes von HIV erforderlich ist, als Therapeutika auf dem Markt oder in fortgeschrittener Erprobung, beispielsweise AZT (Azidothymidin). Diese Verbindung hat jedoch eine Vielzahl von ernsten Nebenwirkungen.

Ein neuerer Ansatz hat zum Ziel, Verbindungen zu finden, die eine Vermehrung des Virus blockieren, indem sie den Zusammenbau infektiöser Viruspartikel behindern. Dies ist dadurch möglich, dass die normale Prozessierung von zur Virusreifung benötigten viralen Proteinen gehemmt wird, indem man bestimmte Chemotherapeutika einsetzt.

HIV-1 und HIV-2 weisen jeweils in ihrem Genom einen Bereich auf, der für eine "HIV-Protease" kodiert. Diese "HIV-Protease" ist für die korrekte proteolytische Spaltung der Vorläuferproteine verantwortlich, die aus den für die "Group Specific Antigens" (gag) und viralen Enzyme (pol) kodierenden Genomabschnitten hervorgehen. Hierbei werden unter anderem die Strukturproteine des Viruskerns, englisch "Core", freigesetzt. Die "HIV-Protease" selbst ist Bestandteil des durch den pol-Genomabschnitt von HIV-1 und HIV-2 kodierten Vorläuferproteines, das auch die Abschnitte für die "Reverse Transcriptase" und die "Integrase" enthält und vermutlich autoproteolytisch gespalten wird.

Die "HIV-Protease" spaltet das Hauptkernprotein ("Major Core Protein") p24 von HIV-1 und HIV-2 bevorzugt N-terminal von Prolinresten, z. B. in den bivalenten Radikalen Phe-Pro, Leu-Pro oder Tyr-Pro. Es handelt sich um eine Protease mit einem katalytisch aktiven Aspartatrest im aktiven Zentrum, eine sogenannte Aspartatprotease.

Aufgrund der zentralen Rolle der "HIV-Protease" bei der Prozessierung der genannten "Core-Proteine" wird davon ausgegangen, dass eine wirksame Inhibierung dieses Enzyms in vivo den Zusammenbau reifer Virionen unterbindet, so dass entsprechende Inhibitoren therapeutisch eingesetzt werden können.

Voraussetzung für die therapeutische Wirksamkeit in vivo ist das Erreichen einer guten Hemmung der Virusvermehrung in Zellversuchen und eine gute Bioverfügbarkeit, z. B. eines hohen Blutspiegels, um so im Körper an infizierten Zellen ausreichend hohe Konzentrationen zu erreichen.

Das Ziel der hier vorliegenden Erfindung ist, neue Hemmstoffe von retroviralen Proteasen, insbesondere der HIV-1-Aspartatprotease, zugänglich zu machen, welche ein von einer derartigen Protease, insbesondere der HIV-1-Protease, nicht spaltbares Peptidisoster enthalten, im Zellversuch schon bei niedrigen Konzentrationen hochwirksam sind und bei oraler oder parenteraler Gabe im Blut solche Konzentrationen erreichen, dass aufgrund der im Zellversuch ermittelten wirksamen Konzentrationen Wirksamkeit gegen Retroviren, vorzugsweise HIV, insbesondere HIV-1, in vivo zu erwarten ist. Diese Verbindungen sind somit als Therapeutika der genannten Erkrankungen anzusehen.

Bei den erfindungsgemässen Verbindungen handelt es sich um Verbindungen der Formel I,

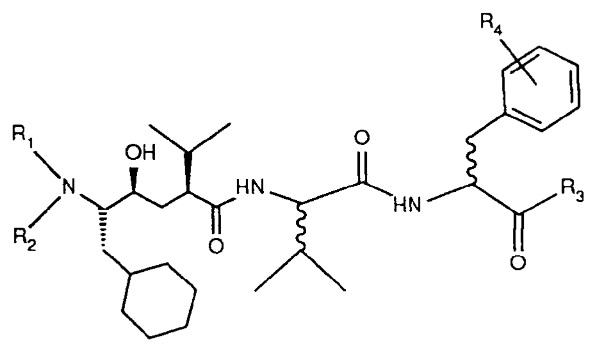

(I),

worin $R_1$ Wasserstoff oder Niederalkyl, $R_2$ Wasserstoff, Niederalkyl, Niederalkoxycarbonyl, Phenyl- oder Naphthyl-niederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu dem bindenden Stickstoffatom ein oder mehrere weitere Heteroatome ausgewählt aus unsubstituiertem oder durch einen $C_1$-$C_4$-Alkylrest substituiertem NH, O, S, S=O oder $SO_2$ als Ringglied enthält, Niederalkanoyl, Phenyl- oder Naphthylniederalkanoyl oder Niederalkansulfonyl bedeutet, $R_3$ Morpholino, Thiomorpholino, S-Oxothiomorpholino oder S,S-Dioxothiomorpholino wobei der Rest Heterocyclyl in Heterocyclylcarbonyl $R_2$ dieselbe Bedeutung wie $R_3$ oder eine von $R_3$ unterschiedliche Bedeutung hat, und $R_4$ Wasserstoff, Hydroxy, $C_1$-$C_4$-Alkoxy, Cyano, Trifluormethyl oder Fluor bedeuten, sowie Salze dieser Verbindungen, sofern salzbildende Gruppen vorliegen.

In der Beschreibung der vorliegenden Erfindung bedeutet der bei der Definition von Gruppen oder Resten, z.B. Niederalkyl, Niederalkoxycarbonyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten und bei Vorliegen von drei oder mehr C-Atomen geradkettig oder verzweigt sein können.

Asymmetrische Kohlenstoffatome in den Substituenten $R_1$ oder $R_2$ oder die in Formel I durch wellenförmige Bindungen mit ihren Substituenten verknüpften Kohlenstoffatome liegen unabhängig voneinander in der (R)-, (S)- oder (R,S)-Konfiguration vor. Somit können die vorliegenden Verbindungen als Isomerengemische oder als reine Isomeren, insbesondere als Diastereomerengemische, Enantiomerenpaare oder reine Enantiomere vorliegen.

Die in der Beschreibung der vorliegenden Erfindung verwendeten allgemeinen Ausdrücke und Bezeichnungen haben vorzugsweise die folgenden Bedeutungen, wobei von den vor-oder nachstehend aufgeführten Definitionen von Resten beliebige Kombinationen oder Einzelreste anstelle der allgemeinen Definitionen eingesetzt werden können:

Niederalkyl $R_1$ oder $R_2$ bedeutet einen geradkettigen oder verzweigtkettigen Rest, z. B. Methyl, Ethyl, n-Propyl, Isopropyl, n-Butyl, sec-Butyl, tert-Butyl, Pentyl, wie Isopentyl, Neopentyl, Hexyl, wie n-Hexyl, oder Heptyl, wie n-Heptyl, wobei $R_1$ und $R_2$ verschieden oder gleich sein können. Bevorzugt ist $R_1$ Wasserstoff und $R_2$ Niederalkyl, insbesondere Methyl, Ethyl oder tert-Butyl, oder Niederalkyl $R_1$ und $R_2$ sind gleich, z. B. Methyl oder Ethyl.

Niederalkoxycarbonyl $R_2$ enthält vorzugsweise einen verzweigten Niederalkylrest, insbesondere einen sec- oder tert-Niederalkylrest, und ist z. B. Butoxycarbonyl, wie tert-Butoxycarbonyl oder Isobutoxycarbonyl. Besonders bevorzugt ist tert-Butoxycarbonyl.

Phenylniederalkoxycarbonyl $R_2$ enthält vorzugsweise einen endständig an den Niederalkylrest gebundenen Phenylrest und ist z. B. Benzyloxycarbonyl.

Naphthylniederalkoxycarbonyl $R_2$ enthält vorzugsweise einen endständig an den Niederalkylrest gebundenen Naphthylrest und ist z. B. 1- oder 2-Naphthyloxycarbonyl.

Heterocyclyl in Heterocyclylcarbonyl $R_2$, welches 5 oder 6 Ringatome enthält, gesättigt ist und über ein Ringstickstoffatom an die Carbonylgruppe gebunden ist und zusätzlich zu dem bindenden Stickstoffatom ein oder mehrere, vorzugsweise bis zu 3, weitere Heteroatome ausgewählt aus unsubstituiertem oder durch einen $C_1$-$C_4$-Alkylrest substituiertem NH, O, S, S=O oder $SO_2$ als Ringglied enthält, ist vorzugsweise Imidazol-1-yl, Piperazino, N-$C_1$-$C_4$-Alkyl-piperazino, z. B. N-Methyl oder N-Ethylpiperazino, N-gebundenes Triazolyl, z. B. N-gebundenes 1,2,3- oder 1,2,4-Triazolyl, N-gebundenes Tetrazolyl, z. B. Tetrazol-1-yl oder -2-yl, Morpholino, Thiomorpholino, S-Oxothiomorpholino oder S,S-Dioxothiomorpholino. Besonders bevorzugt als Heterocyclylcarbonyl $R_2$ ist Morpholinocarbonyl.

Niederalkanoyl $R_2$ ist verzweigt oder unverzweigt und ist z. B. Acetyl, Propionyl oder n-Butyryl, ferner Pivaloyl, Hexanoyl oder Heptanoyl.

Phenylniederalkanoyl $R_2$ enthält Niederalkanoyl, wie unter Niederalkanoyl $R_2$ definiert, welches vorzugs-

weise endständig durch Phenyl substituiert ist, und ist z. B. Phenylacetyl und ferner Benzoyl.

Naphthylniederalkanoyl $R_2$ enthält Niederalkanoyl, wie unter Niederalkanoyl $R_2$ definiert, welches vorzugsweise endständig durch 1-oder 2-Napthyl substituiert ist, z. B. 1- oder 2-Naphthylacetyl oder 1- oder 2-Naphthoyl.

Niederalkansulfonyl $R_2$ enthält Niederalkyl, wie oben für $R_1$ oder $R_2$ definiert, und ist z. B. Methan- oder Ethan-sulfonyl.

$R_3$ ist Morpholino, Thiomorpholino, S-Oxothiomorpholino oder S,S-Dioxothiomorpholino, vorzugsweise Morpholino oder Thiomorpholino. Besonders bevorzugt als Heterocyclyl $R_3$ ist Morpholino.

Der Rest Heterocyclyl in Heterocyclylcarbonyl $R_2$ ist vorzugsweise gleich $R_3$, kann aber auch verschieden sein.

Der Substituent $R_4$ ist in o-, m- oder p-Stellung an den Phenylring gebunden, vorzugsweise in o- oder p-Stellung, insbesondere in p-Stellung, und bedeutet in einer bevorzugten Ausführungsform Wasserstoff, $C_1$-$C_4$-Alkoxy, Cyano oder Fluor, vorzugsweise Wasserstoff.

$C_1$-$C_4$-Alkoxy $R_4$ ist vorzugsweise Methoxy oder Ethoxy.

Salze von Verbindungen der Formel I sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, bei dosisgerechter Anwendung nichttoxischen Salze von Verbindungen der Formel I.

Solche Salze werden beispielsweise von Verbindungen der Formel I mit einer oder mehreren sauren Gruppen, z. B. einer Carboxygruppe, gebildet und sind beispielsweise deren Salze mit geeigneten Basen, wie nichttoxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumverbindungen gebildet werden, z. B. mit N-Methyl-N-ethylamin, Diethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tert-butylamin oder Tris(hydroxymethyl)-methylamin, N,N-Diniederalkyl-N-(hydroxyniederalkyl)-aminen, wie N,N-Dimethyl-N-(2-hydroxyethyl)-amin oderTris-(2-hydroxyethyl)amin, N-Methyl-D-glucamin oder quaternären Ammoniumsalzen, wie Tetrabutylammoniumsalzen. Die Verbindungen der Formel I mit einer oder mehreren basischen Gruppen, z.B. einer Amino- oder Iminogruppe, können Säureadditionssalze bilden, beispielsweise mit anorganischen Säuren, z. B. Halogenwasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie, z. B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z. B. Glutaminsäure oder Asparaginsäure, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung zeigen Hemmwirkung auf retrovirale Aspartatproteasen, insbesondere HIV-Protease-hemmende Wirkungen. In erster Linie hemmen sie in den nachfolgend beschriebenen Tests in Konzentrationen von mit einer $IC_{50}$ von 10 bis 1000 nM, insbesondere von 10 bis 100 nM, die Wirkung der HIV-Protease von HIV-1 und sind daher geeignete Mittel gegen durch diese oder verwandte Retroviren verursachte Krankheiten, wie gegen AIDS.

Die Fähigkeit der Verbindungen der Formel I, die proteolytische Aktivität von z. B. HIV-1 Protease zu inhibieren, lässt sich beispielsweise gemäss dem von A. D. Richards et al., J. Biol. Chem. 265(14), 7733-7736 (1990), beschriebenen Verfahren demonstrieren. Hierbei wird als Substrat für eine rekombinante HIV-1-Protease (Herstellung gemäss Billich, S., et al., J. Biol. Chem. 263(34), 17905 - 17908 (1990)) ein synthetisches chromophores Peptid (z. B. HKARVL[$NO_2$]FEANleS (Bachem, Schweiz; vgl. M. W. Pennington et al., Peptides 1990, ed.: E. Girault and D. Andrew (1991), ESCOM Sci. Publ. B.V., S. 787-789) oder ein Icosapeptid wie RRSNQVSQNYPIVQNIQGRR (hergestellt durch Peptidsynthese nach bekannten Verfahren: J. Schneider et al., Cell 54, 363-368 (1988)) eingesetzt, das einer der Spaltstellen des gag-Vorläuferproteins entspricht. Dieses Substrat und Spaltprodukte davon können durch Hochdruckflüssig-Chromatographie (HPCL) gemessen werden.

Beispielsweise wird ein zu testender Hemmstoff der Formel I in Dimethylsulfoxid gelöst; der Enzymtest wird durchgeführt, indem geeignete Verdünnungen des Hemmstoffes in 20 mM β-Morpholinoethansulfonsäure (MES)-Puffer pH 6,0 zum Assay-Mix aus 67,2 μM des oben genannten chromophoren Peptides in 0,3 M Natriumacetat, 0,1 M NaCl pH 7,4 oder 122 μM des oben genannten Icosapeptids in 20 mM MES-Puffer pH 6,0 zugegeben werden. Die Grösse der Ansätze beträgt jeweils 100 μl. Die Reaktion wird gestartet durch Zugabe von im ersten Fall 2 μl, im zweiten Fall 10 μl HIV-I-Protease und im ersten Fall nach 15 min durch Zugabe von 100 μl 0,3 M $HClO_4$, im zweiten Fall nach einer Stunde Inkubation bei 37 °C durch Zugabe von 10 μl 0,3 M $HCLO_4$ gestoppt. Die Reaktionsprodukte werden nach Abzentrifugieren der Probe für 5 min bei 10 000 x g in 100 μl (Ansatz mit chromophorem Peptid) bzw. 20 μl (Icosapeptid-Ansatz) des erhaltenen Überstandes und nach Auftragen auf eine 125 x 4,6 mm Nucleosil® C18-5μ-HPLC-Säule (Macherey & Nagel, Düren) und Elution quantifiziert anhand der Peak-Höhe des Spaltproduktes bei 280 (Ansatz mit chromophorem Peptid) oder bei 215 nm (Ansatz mit Icosapeptid), Gradient: 100 % El.1->50 % El.2/50 % El.2 (El.1: 75 % Acetonitril, 90 % $H_2O$, 0,1 % Trifluoressigsäure (TFA); El.2: 75 % Acetonitril, 25 % $H_2O$, 0,08 % TFA) innerhalb von 15 min; Durchflussrate 1 ml/min.

Hierbei werden für Verbindungen der Formel I vorzugsweise $IC_{50}$-Werte ($IC_{50}$ = Konzentration, welche die Aktivität der HIV-1-Protease gegenüber einer Kontrolle ohne Hemmstoff um 50 % senkt) von etwa $10^{-8}$ bis $10^{-6}$M, insbesondere von $10^{-8}$ bis $10^{-7}$M, ermittelt. In einem weiteren Test kann gezeigt werden, dass die Verbindungen der vorliegenden Erfindung Zellen, die normalerweise von HIV infiziert werden, vor einer solchen Infektion schützen oder zumindest eine solche Infektion verlangsamen. Dabei wird die menschliche T-Zell-Leukämie Zellinie MT-2 (Science 229, 563 (1985)), die empfindlich gegen den zytopathogenen Effekt von HIV ist, mit HIV-1 allein oder mit HIV-1 in Gegenwart einer der erfindungsgemässen Verbindungen inkubiert und nach einigen Tagen die Lebensfähigkeit der so behandelten Zellen beurteilt. Hierzu werden die MT-2-Zellen in RPMI 1640-Medium (Gibco, Schweiz; RPMI 1640 enthält ein Aminosäurengemisch ohne L-Gln), das mit 10% hitzeinaktiviertem fetalem Kälberserum, L-Glutamin, Hepes (2-[4-(2-Hydroxyethyl)-1-piperazino]-ethansulfonsäure) und Standardantibiotika supplementiert ist, bei 37 °C in befeuchteter Luft mit 5% $CO_2$ gehalten. 50 μl der jeweiligen Testverbindung in Kulturmedium und 100 μl HIV-1 in Kulturmedium (800 TCID50/ml) (TCID50=Tissue Culture Infectious Dose 50 = Dosis, die 50% der MT-2-Zellen infiziert) werden zu $4x10^3$ exponentiell wachsenden MT-2-Zellen in 50 μl Kulturmedium pro Vertiefung auf 96-Loch-Mikrotiterplatten gegeben. Parallele Ansätze auf einer weiteren Mikrotiterplatte mit Zellen und Testverbindung erhalten 100 μl Kulturmedium ohne Virus. Nach 4 Tagen Inkubation wird in 10 μl Zellüberstand die Reverse-Transkriptase (RT)-Aktivität ermittelt. Die RT-Aktivität wird bestimmt in 50 mM Tris (α,α,α-Tris(hydroxymethyl)-methylamin, Ultra pur, Merck, Bundesrepublik Deutschland) pH 7,8; 75 mM KCl, 2 mM Dithiothreitol, 5 mM $MgCl_2$; 0,05% Nonidet P-40 (Detergens; Sigma, Schweiz); 50 μg/ml Polyadenylic Acid (Pharmacia, Schweden); 1,6 μg/ml dT(12-18) (Sigma, Schweiz). Die Mischung wird durch einen 0,45 μ Acrodisc-Filter (Gelman Sciences Inc, Ann Arbor) abfiltriert und bei -20°C aufbewahrt. Zu Aliquoten dieser Lösung werden 0,1% (v/v) [alpha-$^{32}$P]dTTP zum Erzielen einer radioaktiven Endaktivität von 10 μCi/ml zugegeben. 10 μl des Kulturüberstandes werden auf eine neue 96-Loch-Mikrotiterplatte übertragen und hierzu 30 μl des genannten RT-Cocktails gegeben. Nach Mischen wird die Platte für 1,5 bis 3 h bei 37 °C inkubiert. 5 μl dieser Reaktionsmischung werden auf DE81-Papier (Whatman) überführt. Die getrockneten Filter werden 3-mal für 5 min mit 300 mM NaCl/25 mM Tri-Natriumcitrat und 1-mal mit 95% Ethanol gewaschen und erneut luftgetrocknet. Die Auswertung erfolgt in einem Matrix Packard 96well counter (Packard, Zürich, Schweiz). Die ED90-Werte werden errechnet und sind als die niedrigste Konzentration der jeweiligen Testverbindung definiert, welche die RT-Aktivität um 90% im Vergleich zu nicht mit der Testsubstanz behandelten Zellansätzen senkt. Die RT-Aktivität ist dabei ein Mass für die HIV-1-Vermehrung.

Die erfindungsgemässen Verbindungen zeigen hierbei vorzugsweise eine ED90 von $10^{-8}$ bis $10^{-6}$M, insbesondere von $10^{-8}$ bis $10^{-7}$M.

Die Verbindungen der vorliegenden Erfindung zeigen vorteilhafte pharmakokinetische Eigenschaften, die erwarten lassen, dass sie in vivo die genannten Hemmwirkungen entfalten. So ist beispielsweise der Blutspiegel bei intravenöser oder intraperitonealer Applikation von 20 mg/kg einer der genannten Verbindungen an Mäusen 1 h nach der Applikation etwa gleich oder höher als die ED90 im Zellassay.

Bei peroraler Gabe von 120 mg/kg einer der genannten Verbindungen lassen sich 30 min nach der Applikation Konzentrationen im Mäuseblut finden, die ebenfalls über der ED90 im Zellassay liegen, vorzugsweise etwa das zehnfache der ED90 im Zellassay ausmachen.

Die Ermittlung des Blutspiegels wird beispielsweise wie folgt vorgenommen: Die zu untersuchenden Verbindungen werden in einem Lösungsmittel, wie DMSO (Dimethylsulfoxid), gelöst. Eine Lösung von Hydroxypropyl-β-cyclodextrin (20 % w/v) in Wasser wird zugegeben bis zum Erhalt der gewünschten Konzentration des Wirkstoffes (beispielsweise 2 mg/ml für parenterale Applikation, 12 mg/ml für orale Applikation) bei gleichzeitiger Einstellung einer Konzentration von 5 % DMSO (v/v). Verbindungen, die unter diesen Bedingungen unlöslich sind, werden nur intraperitoneal appliziert, lösliche Verbindungen zusätzlich intravenös. Nach

Applikation der Verbindungen (beispielsweise 20 mg/kg intravenös oder intraperitoneal, oder 120 mg/kg peroral) wird zu verschiedenen Zeitpunkten, z. B. nach 30 oder 60 min, Blut entnommen. Pro Zeitpunkt wird das Blut von drei Mäusen verwendet und entweder für jede Maus einzeln oder aus dem vereinten Blut der drei Mäuse nach Zugabe eines Lösungsmittels, z. B. Acetonitril, und anschliessender Zentrifugation der Überstand gewonnen. Die Konzentration des Wirkstoffes wird mittels HPLC gemessen, beispielsweise auf einer Nucleosil® $5C_{18}$-Säule (Macherey-Nagel) von 120 mm Länge und 4,6 mm Durchmesser mit entweder 60 % Acetonitril/40 % Wasser/0,05 %Trifluoressigsäure (v/v) oder 50 % Acetonitril/40 % Wasser/0,05 % Trifluoressigsäure (v/v) als Laufmittel (Flussrate 1 ml/min) durch Detektion und Quantifikation bei 200 nm.

Bei den im folgenden genannten Gruppen von Verbindungen der Formel I können in sinnvoller Weise, z. B. zur Ersetzung allgemeinerer durch speziellere Definitionen, Definitionen von Resten aus den oben genannten allgemeinen Definitionen eingesetzt werden.

Bevorzugt sind Verbindungen der Formel I, worin $R_1$ Wasserstoff bedeutet, $R_2$ Wasserstoff, Niederalkoxycarbonyl, insbesondere sec- oder tert-Niederalkyl-oxycarbonyl, z. B. Butoxycarbonyl, wie tert-Butoxycarbonyl oder Isobutoxycarbonyl, besonders bevorzugt tert-Butoxycarbonyl, oder Heterocyclylcarbonyl bedeutet, worin Heterocyclyl 5 oder 6 Ringatome enthält, gesättigt ist und über ein Ringstickstoffatom an die Carbonylgruppe gebunden ist und zusätzlich zu dem bindenden Stickstoffatom unsubstituiertes oder durch einen $C_1$-$C_4$-Alkylrest substituiertes NH, O, S, S=O oder $SO_2$ als Ringglied enthält, insbesondere Piperazinocarbonyl, N-$C_1$-$C_4$-Alkyl-piperazinocarbonyl, z. B. N-Methyl oder N-Ethylpiperazinocarbonyl, Morpholinocarbonyl, Thiomorpholinocarbonyl, S -Oxothiomorpholinocarbonyl oder S,S-Dioxothiomorpholinocarbonyl, besonders bevorzugt Morpholinocarbonyl, $R_3$ Morpholino, Thiomorpholino, S-Oxothiomorpholino oder S,S -Dioxothiomorpholino bedeutet, insbesondereMorpholino oder Thiomorpholino, besonders bevorzugt Morpholino, und $R_4$ Wasserstoff bedeutet, oder pharmazeutisch verwendbare Salze von solchen Verbindungen, die salzbildende Gruppen enthalten.

Stärker bevorzugt sind die Verbindungen der Formel Ib,

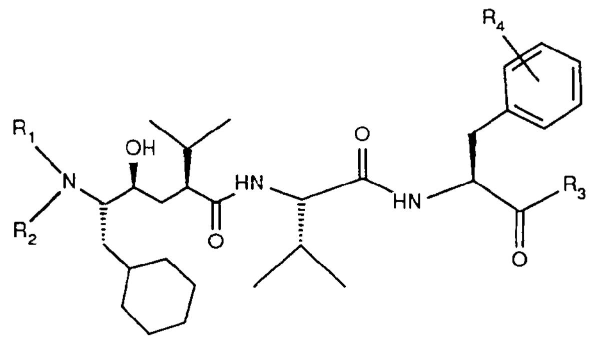

(Ib)

worin die Reste $R_1$, $R_2$, $R_3$ und $R_4$ die bisher für Verbindungen der Formel I genannten Bedeutungen haben, oder pharmazeutisch verwendbare Salze von solchen Verbindungen, die salzbildende Gruppen enthalten.

Besonders bevorzugt sind die Verbindungen der Formel Ib, worin $R_1$ Wasserstoff bedeutet, $R_2$ Wasserstoff, Niederalkoxycarbonyl, insbesondere sec- oder tert-Niederalkyl-oxycarbonyl, z. B. Butoxycarbonyl, wie tert-Butoxycarbonyl oder Isobutoxycarbonyl, besonders bevorzugt tert-Butoxycarbonyl, oder Heterocyclylcarbonyl bedeutet, worin Heterocyclyl 5 oder 6 Ringatome enthält, gesättigt ist und über ein Ringstickstoffatom an die Carbonylgruppe gebunden ist und zusätzlich zu dem bindenden Stickstoffatom unsubstituiertes oder durch einen $C_1$-$C_4$-Alkylrest substituiertes NH, O, S, S=O oder $SO_2$ als Ringglied enthält, insbesondere Piperazinocarbonyl, N-$C_1$-$C_4$-Alkyl-piperazinocarbonyl, z. B.

N-Methyl oder N-Ethylpiperazinocarbonyl, Morpholinocarbonyl, Thiomorpholinocarbonyl, S-Oxothiomorpholinocarbonyl oder S,S-Dioxothiomorpholinocarbonyl, besonders bevorzugt Morpholinocarbonyl, $R_3$ über ein Ringstickstoffatom gebundenes Heterocyclyl bedeutet, wie oben bei Heterocyclylcarbonyl $R_3$ definiert, insbesondere Piperazino, N-$C_1$-$C_4$-Alkyl-piperazino, z. B. N-Methyl oder N-Ethylpiperazino, Morpholino, Thiomorpholino, S-Oxothiomorpholino oder S,S-Dioxothiomorpholino, besonders bevorzugt Morpholino, und $R_4$ Wasserstoff bedeutet, oder pharmazeutisch verwendbare Salze von solchen Verbindungen, die salzbildende Gruppen enthalten.

Noch stärker bevorzugt sind die Verbindungen der Formel Ib, worin $R_1$ Wasserstoff bedeutet, $R_2$ Wasserstoff, sec- oder tert-Niederalkyl-oxycarbonyl, Piperazinocarbonyl, N-$C_1$-$C_4$-Alkyl-piperazinocarbonyl, Morpholinocarbonyl, Thiomorpholinocarbonyl, S-Oxothiomorpholinocarbonyl oder S,S-Dioxothiomorpholinocarbonyl bedeutet, $R_3$ Morpholino, Thiomorpholino, S-Oxothiomorpholino oder S,S-Dioxothiomorpholino, insbesondere Morpholino oder Thiomorpholino, besonders bevorzugt Morpholino, bedeutet und $R_4$ Wasserstoff bedeutet,

oder pharmazeutisch verwendbare Salze von solchen Verbindungen, die salzbildende Gruppen enthalten.

Sehr bevorzugt sind die Verbindungen der Formel Ib, worin $R_1$ Wasserstoff, $R_2$ Niederalkoxycarbonyl, insbesondere sec- oder tert-Niederalkyl-oxycarbonyl,wie tert-Butoxycarbonyl oder Isobutoxycarbonyl, bedeutet, $R_3$ Morpholino bedeutet und $R_4$ Wasserstoff bedeutet.

In allererster Linie betroffen sind die in den Beispielen genannten Verbindungen oder die pharmazeurisch verwendbaren Salze dieser Verbindungen, sofern salzbildende Gruppen vorliegen.

Die Verbindungen der Formel I und Salze von solchen Verbindungen mit mindestens einer salzbildenden Gruppe werden nach an sich bekannten Verfahren erhalten, z. B. indem man

a) zur Herstellung von Verbindungen der Formel I, worin wenigstens einer der Reste $R_1$ oder $R_2$ Niederalkyl bedeutet und der andere Wasserstoff bedeutet oder $R_1$ und $R_2$ beide Niederalkyl bedeuten und die übrigen Reste die für Verbindungen der Formel I genannten Bedeutungen haben, eine Aminoverbindung der Formel II (welche einer Verbindung der Formel I entspricht, in der $R_1$ und $R_2$ Wasserstoff bedeuten),

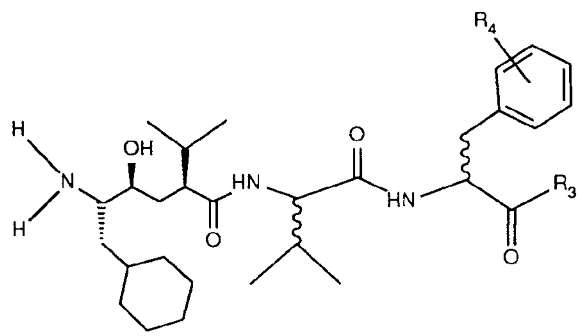

(II)

worin die $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, mit einem Alkylierungsreagenz umsetzt zur Einführung des Restes Niederalkyl $R_1$, des Restes Niederalkyl $R_2$ oder beider Reste Niederalkyl $R_1$ und Niederalkyl $R_2$, und vorhandene Schutzgruppen abspaltet, oder

b) zur Herstellung einer Verbindung der Formel I, worin $R_2$ Niederalkoxycarbonyl, Phenyl- oder Naphthylniederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu dem bindenden Stickstoffatom unsubstituiertes oder durch einen $C_1$-$C_4$-Alkylrest substituiertes NH, O, S,S=O oder $SO_2$ als Ringglied enthält, Niederalkanoyl, Phenyl- oder Naphthylniederalkanoyl oder Niederalkansulfonyl bedeutet, und die übrigen Reste die genannten Bedeutungen haben, und worin freie funktionelle Gruppen mit Ausnahme der an der Reaktion beteiligten erforderlichenfalls in geschützter Form vorliegen können, eine Aminoverbindung der Formel IV,

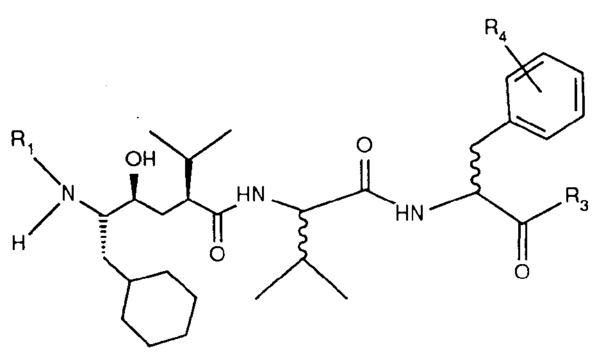

(IV)

worin die Reste $R_1$, $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, oder ein reaktionsfähiges Aminoderivat hiervon, mit einer Säure der Formel V,

$R_2$-OH (V)

worin $R_2$ Niederalkoxycarbonyl, Phenyl- oder Naphthyl-niederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu dem bindenden Stickstoffatom unsubstituiertes oder durch einen

$C_1$-$C_4$-Alkylrest substituiertes NH, O, S, S=O oder $SO_2$ als Ringglied enthält, Niederalkanoyl, Phenyl- oder Naphthylniederalkanoyl oder Niederalkansulfonyl bedeutet, und worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet, oder
c) eine Aminoverbindung der Formel VI,

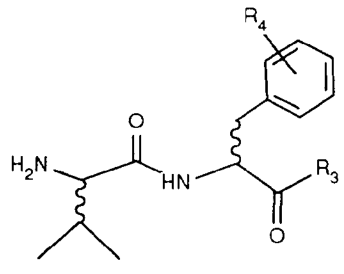

(VI)

worin $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, oder ein reaktionsfähiges Aminoderivat davon, mit einer Carbonsäure der Formel VII,

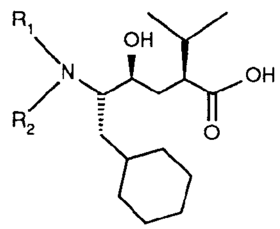

(VII)

worin die Reste $R_1$ und $R_2$ die für Verbindungen der Formel I genannten Bedeutungen haben, und worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet, oder
d) eine Aminoverbindung der Formel VIII,

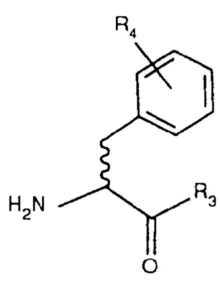

(VIII)

worin $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, oder ein reaktionsfähiges Aminoderivat davon, mit einer Carbonsäure der Formel IX,

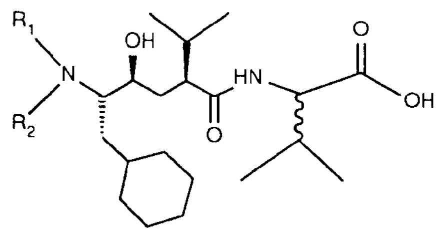

(IX)

worin $R_1$ und $R_2$ die für Verbindungen der Formel I genannten Bedeutungen haben, und worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet, oder

e) eine Verbindung der Formel X,

$R_3$-H (X)

worin $R_3$ die für Verbindungen der Formel I genannten Bedeutungen hat, und worin freie funktionelle Gruppen mit Ausnahme der an der Reaktion beteiligten erforderlichenfalls in geschützter Form vorliegen können, oder ein reaktionsfähiges Derivat davon, mit einer Carbonsäure der Formel XI

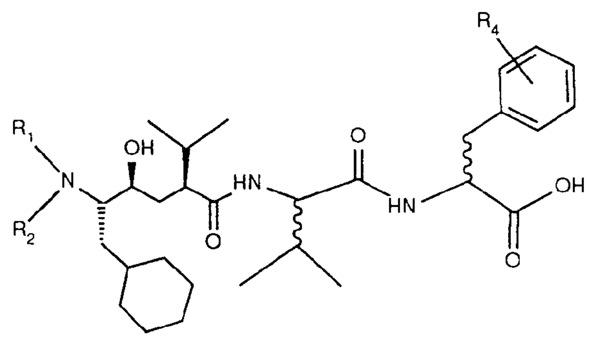

(XI)

worin $R_1$, $R_2$ und $R_4$ die für Verbindungen der Formel I genannten Bedeutungen haben, worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet,

und gewünschtenfalls eine nach einem der vorstehenden Verfahren a) bis e) erhaltene Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel I in eine andere erfindungsgemässe Verbindung der Formel I umwandelt.

Verfahren a) (Nukleophile Substitution)

In Ausgangsmaterialien der Formel II sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Ein Alkylierungsreagenz zur Einführung des Restes Niederalkyl $R_1$, des Restes Niederalkyl $R_2$ oder beider Reste Niederalkyl $R_1$ und Niederalkyl $R_2$, ist insbesondere ausgewählt aus den folgenden Reagenzien:

Alkylierungsmittel sind in der Deutschen Offenlegungsschrift 2 331133 genannt, welche man unter den dort genannten Reaktionsbedingungen mit einer Verbindung der Formel I umsetzen kann.

Weitere Alkylierungsmittel sind ausgewählt aus entsprechenden Niederalkylverbindungen der Formel III,

R-Y (III)

worin R einen $R_1$ und/oder $R_2$ entsprechenden Niederalkylrest bedeutet Y eine Abgangsgruppe ist. Eine Abgangsgruppe ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Iodwasserstoffsäure, oder mit einer starken organischen Sulfonsäure, wie einer gegebenenfalls, z. B. durch Halogen, wie Fluor, substituierten Niederalkansulfonsäure

oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure, verestertes Hydroxy oder mit Stickstoffwasserstoffsäure verestertes Hydroxy.

Die Reaktion kann ünter den Bedingungen einer nukleophilen Substitution erster oder zweiter Ordnung ablaufen.

Beispielsweise wird eine Verbindung der Formel III in einem polaren aprotischen Lösungsmittel, z. B. Aceton, Acetonitril, Nitromethan, Dimethylsulfoxid, Hexamethylphosphorsäuretriamid oder Dimethylformamid, mit der Aminoverbindung der Formel II umgesetzt, oder die Reaktion wird in einem protischen Lösungsmittel, z.B. Alkoholen, wie Methanol, Ethanol, Ethylenglykol oder Isopropanol, oder Wasser, dem gegebenenfalls als Lösungsvermittler organische Lösungsmittel, z. B. Ethanol, Tetrahydrofuran oder Aceton, beigemischt sind, durchgeführt. Die Reaktion wird in Ab- oder Anwesenheit von Katalysatoren, wie Lewissäuren, z. B. $SnCl_4$, $BF_3$, $AlCl_3$, $FeCl_3$, $ZnCl_2$ oder $SbCl_5$ durchgeführt, wobei die genannten Lewissäuren vorzugsweise dann Verwendung finden, wenn Y ein Halogenatom ist. Die Substitutionsreaktion wird gegebenenfalls bei erniedrigter oder erhöhter Temperatur, z. B. in einem Temperaturbereich von etwa -40 ° bis etwa 120 °C, bevorzugt von etwa -10 ° bis etwa 100 °C, und gegebenenfalls unter Inertgas, z. B. Stickstoff- oder Argonatmosphäre, durchgeführt.

Die Reaktion kann auch so durchgeführt werden, dass der Reihe nach zwei verschiedene Verbindungen der Formel III eingesetzt werden, so dass Verbindungen der Formel I hergestellt werden, in denen $R_1$ und $R_2$ verschiedene Niederalkylreste bedeuten. Dies wird beispielsweise erreicht durch Einsatz geeigneter stöchiometrischer Mengen der Ausgangsverbindungen, z. B. bei der Einführung des ersten Niederalkylrestes durch Einsatz einer molaren Menge der ersten Verbindung der Formel III, die unter der molaren Menge der Verbindung der Formel II liegt, oder durch Verwendung geeigneter Aminoschutzgruppen, die nach der ersten Alkylierung abgespalten werden, und anschliessende Alkylierung mit einer zweiten Verbindung der Formel III, die einen anderen Niederalkylrest als die erste Verbindung trägt.

Zu den Schutzgruppen für funktionelle Gruppen in Ausgangsmaterialien, deren Umsetzung vermieden werden soll, insbesondere Carboxy-, Amino-, Imino- oder Hydroxygruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen, und dass sie nicht in den Endprodukten vorliegen. Verbindungen der Formel I mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl, oder sec-Niederalkoxycarbonyl, z. B. Isobutoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di-oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, Fluorenylmethoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1 -Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl oder insbesondere tert-Butyl-dimethylsilyloxycarbonyl. Das Siliciumatom der Silyloxycarbonylgruppe kann auch durch zwei Niederalkyl-, z. B. Methylgruppen, und eine Amino- oder Carboxygruppe eines zweiten Moleküls einer zu schützenden Verbindung substituiert sein. Verbindungen mit solchen Schutzgruppen lassen sich z. B. mit Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl, Triniederalkylsilyloxycarbonyl, z. B. tert-Butyldimethylsilyloxycarbonyl, oder eine in Form eines Lactons, insbesondere eines γ-Lactons, geschützte Carboxygruppe.

Eine geschützte Aminogruppe ist durch eine Aminoschutzgruppe geschutzt, z. B. in Form einer Acylamino-, Arylmethylamino-, veretherten Mercaptoamino-, 2-Acyl-niederalk-1enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylniederalkoxycarbonyl, z. B. Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert-Butyl, Niederalkoxy, wie Methoxy, Halogen, wie Chlor, und/oder Nitro substituiertes Phenyl ist, z. B. als 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als Protonendonatoren kommen in erster Linie stark anorganische Säuren, wie Schwefelsäure, Phosphorsäure oder Halogenwasserstoffsäuren, z. B. das Chlor- oder Bromwasserstoffsäure, oder organische Sulfonsäuren, wie p-Toluolsulfonsäure, in Frage.

Bevorzugte Aminoschutzgruppen sind Niederalkoxycarbonyl, Phenylniederalkoxycarbonyl, 9-Fluorenylniederalkoxycarbonyl, 2-Niederalkanoyl-niederalk-1-en-2-yl oder Niederalkoxycarbonyl-niederalk-1-en-2-yl, besonders bevorzugt tert-Butoxycarbonyl oder Benzyloxycarbonyl.

Zum Schutz von Iminogruppen können alle genannten monovalenten Aminoschutzgruppen und, soweit chemisch möglich, auch zweiwertige Aminoschutzgruppen verwendet werden.

Eine Hydroxygruppe kann beispielsweise durch eine Acylgruppe, z. B. durch Halogen, wie Chlor, substituiertes Niederalkanoyl, wie 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Eine Hydroxygruppe kann auch durch Triniederalkylsilyl, z. B. Trimethylsilyl, Triisopropylsilyl oder insbesondere tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1 -Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein.

Zwei in einem Molekül vorkommende, insbesondere benachbarte Hydroxygruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines $\gamma$-Lactones, geschützt sein.

Vorzugsweise ist eine geschützte Hydroxygruppe durch Triniederalkylsilyl oder als Lacton geschützt, insbesondere durch tert-Butyl-dimethylsilyl oder als $\gamma$-Lacton.

Als Schutzgruppe, beispielsweise Carboxyschutzgruppe, im Sinne dieser Anmeldung ist ausdrücklich auch ein in leicht abspaltbarer Weise mit der zu schützenden funktionellen Gruppe, beispielsweise Carboxygruppe, verbundener polymerer Träger zu verstehen, wie er z. B. für die Merrifield-Synthese geeignet ist. Ein solcher geeigneter polymerer Träger ist insbesondere ein durch Copolymerisation mit Divinylbenzol schwach vernetztes Polystyrolharz, das zur reversiblen Bindung geeignete Brückenglieder trägt.

Die Abspaltung der Schutzgruppen, die nicht Bestandteil des gewünschten Endproduktes der Formel I sind, z. B. der Carboxy-, Amino-, Imino- und/oder Hydroxyschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder mittels anderer Reduktionsmittel, sowie Photolyse, gegebenenfalls stufenweise oder gleichzeitig, wobei auch enzymatische Methoden verwendet werden können. Die Abspaltung der Schutzgruppen ist beispielsweise in den weiter vorn im Abschnitt über "Schutzgruppen" genannten Standardwerken beschrieben.

So kann beispielsweise geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls

substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-dithionit, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoffabgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy um gewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes-Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z.B. Trimethylsilyloxycarbonyl, geschütztes Carboxy kann in üblicher Weise solvolytisch, z.B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als $\gamma$-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, bevorzugt in polaren Lösungsmitteln, wie Diniederalkylniederalkanoylamiden, z. B. Dimethylformamid, Ethern, wie cyclischen Ethern, z. B. Dioxan, oder Alkoholen, wie Methanol, Ethanol oder Propanol, wobei Methanol besonders bevorzugt ist, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig- oder Trifluoressigsäure, gegebenenfalls in Gegenwart von Wasser, und eine als Silylamino geschützten Aminogruppe z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Auf die selbe Weise kann man direkt an ein Heteroatom, wie Stickstoff, gebundenes Silyl, wie Trimethylsilyl, in Gegenwart von Fluoridionen abspalten.

Die Freisetzung von Iminogruppen erfolgt durch Abspaltung der betreffenden Schutzgruppen in analoger Weise, wie für die Aminoverbindungen beschrieben.

In Form einer Azidogruppe geschütztes Amino wird z. B. durch Reduktion in freies Amino überführt, beispielsweise durch katalytische Hydrierung mit Wasserstoff in Gegen wart eines Hydrierkatalysators, wie Platinoxid, Palladium, Palladium auf Aktivkohle oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol, insbesondere Methanol oder Ethanol, oder Dioxan, bei etwa 20 °C bis 25 °C, oder auch unter Kühlen oder Erwärmen im Bereich von etwa 0 bis 50 °C, bei Wasserstoffdrucken von Normaldruck bis 5 atm, vorzugsweise von etwa 1 atm, durchgeführt.

Eine durch eine geeignete Acylgruppe, eine Triniederalkylsilylgruppe oder durch gegebenenfalls substituiertes 1-Phenylniederalkyl geschützte Hydroxygruppe wird analog einer entsprechend geschützten Aminogruppe freigesetzt. Eine durch 2,2-Dichloracetyl geschützte Hydroxygruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxygruppe durch Acidolyse, z. B. durch Behandeln mit einer Mineralsäure oder einer starken Carbonsäure, z. B. Trifluoressigsäure, freigesetzt. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt.

Die Temperaturen für die Freisetzung der geschützten funktionellen Gruppen liegen vorzugsweise zwischen -80 und 100 °C, besonders bevorzugt zwischen -20 und 50 °C, beispielsweise zwischen 10 und 35°C, wie im Bereich der Raumtemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

<u>Verfahren b)</u> (Kondensation unter Bildung einer Amidbindung)

In Ausgangsmaterialien der Formeln IV und V sind funktionelle Gruppen mit Ausnahme der Gruppen, die an der Reaktion teilnehmen sollen oder unter den Reaktionsbedingungen nicht oder nur in geringem Masse reagieren, unabhängig voneinander durch Schutzgruppen geschützt.

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, oder in H.-D. Jakubke und H. Jescheit, "Aminosäuren, Peptide, Proteine", Verlag Chemie, Weinheim, Deerfield Beach und Basel 1982, beschrieben.

Die Carbonsäuren der Formel V liegen entweder mit freier Carboxygruppe vor, oder als reaktionsfähiges Derivat hiervon, beispielsweise als von der freien Carboxyverbindung abgeleiteter aktivierter Ester, als reaktionsfähiges Anhydrid, oder ferner als reaktionsfähiges cyclisches Amid. Die reaktionsfähigen Derivate können auch in situ gebildet werden und gegebenenfalls nur dort vorliegen.

Aktivierte Ester von Verbindungen der Formel V mit einer Carboxygruppe sind insbesondere am Verknüpfungskohlenstoffatom des veresternden Restes ungesättigte Ester, z. B. vom Vinylester-Typ, wie Vinylester (erhältlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z. B. durch Behandeln der entsprechenden Säure mit einem geeigneten N,N'-disubstituierten Carbodiimid, z. B. N,N'-Dicyclohexylcarbodiimid; Carbodiimid-Methode), oder N,N-disubstituierte Amidinoester (erhältlich z. B. durch Behandeln der entsprechenden Säure mit einem N,N-disubstituierten Cyananmid; Cyanamid-Methode), geeignete Arylester, insbesondere durch elektronenanziehende Substituenten substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, insbesondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro- 1,2,3-benztriazin-4-on, z. B. nach der Anhydrid- oder Carbodiimid-Methode; Methode der aktivierten N-Hydroxyester). Auch innere Ester, z. B. γ-Lactone, sind einsetzbar.

Anhydride von Säuren können symmetrische oder vorzugsweise gemischte Anhydride dieser Säuren sein, z. B. Anhydride mit anorganischen Säuren, wie Säurehalogenide, insbesondere Säurechloride (erhältlich z. B. durch Behandeln der entsprechenden Säure mit Thionylchlorid, Phosphorpentachlorid oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1-Niederalkoxycarbonyl-2-niederalkoxy- 1,2-dihydrochinolin; Methode der gemischten O-Alkylkohlensäureanhydride), oder Anhydride mit dihalogenierter, insbesondere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-Phenylphosphoramidochloridat erhalten kann oder durch Umsetzung von N-Alkylphosphorsäureamiden in Gegenwart von Sulfonsäureanhydriden und/oder racemisierungssenkenden Additiven, wie N-Hydroxybenztriazol, oder in Gegenwart von Cyanphosphonsäurediethylester) oder mit Phosphorigsäurederivaten, oder Anhydride mit organischen Säuren, wie gemischte Anhydride mit organischen Carbonsäuren (erhältlich z. B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z. B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methan- oder p-Toluolsulfonsäurechlorid; Methode der gemischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride).

Geeignete cyclische Amide sind insbesondere Amide mit fünfgliedrigen Diazacyclen aromatischen Charakters, beispielsweise Amide, die mit geeigneten Carbonylverbindungen hergestellt werden können, z. B. Amide mit Imidazolen, z. B. Imidazol (erhältlich z. B. durch Behandeln der entsprechenden Säure mit N,N'-Carbonyldiimidazol; Imidazol-Methode), oder Pyrazolen, z. B. 3,5-Dimethylpyrazol (erhältlich z. B. über das Säurehydrazid durch Behandeln mit Acetylaceton; Pyrazolid-Methode).

Wie erwähnt, können Derivate von Carbonsäuren, die als Acylierungsmittel verwendet werden, auch in situ gebildet werden, häufig sogar ausschliesslich. Diese Methode ist besonders zu bevorzugen. So kann man z. B. N,N'-disubstituierte Amidinoester in situ bilden, indem man das Gemisch des Ausgangsmaterials der Formel IV und der als Acylierungsmittel verwendeten Säure der Formel V in Gegenwart eines geeigneten N,N'-disubstituierten Carbodiimids, z. B. N,N'-Cyclohexylcarbodiimid, zur Reaktion bringt, beispielsweise in Gegenwart einer geeigneten Base, wie Triethylamin. Weiter kann man Amino- oder Amidoester der als Acylierungsmittel verwendeten Säure in Gegenwart des zu acylierenden Ausgangsmaterials der Formel V bilden, indem man das Gemisch der entsprechenden Säure- und Amino-Ausgangsstoffe in Gegenwart eines N,N'-disubstituierten Carbodiimids, z. B. N,N'-Dicyclohexylcarbodiimid, und eines N-Hydroxyamins oder N-Hydroxy-amids,

z. B. N-Hydroxysuccinimid, gegebenenfalls in Anwesenheit einer geeigneten Base, z. B. 4-Dimethylaminopyridin, umsetzt. Ferner kann man durch Umsetzung mit N,N,N',N'-Tetraalkyluroniumverbindungen, wie O-Benztriazol-1-yl-N,N,N',N'-tetra-methyl-uronium-hexafluorphosphat, Aktivierung in situ erreichen. Schliesslich können Phosphorsäureanhydride der Carbonsäuren der Formel V in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

Die Aminogruppe von Verbindungen der Formel IV, die an der Reaktion teilnimmt, trägt vorzugsweise mindestens ein reaktionsfähiges Wasserstoffatom, insbesondere, wenn die damit reagierende Carboxygruppe in reaktionsfähiger Form vorliegt; sie kann aber auch selbst derivatisiert sein, z. B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat einer solchen Verbindung mit einer Aminogruppe ist z. B. auch ein Carbaminsäurehalogenid, wobei die an der Reaktion teilnehmende Aminogruppe durch Halogencarbonyl, z. B. Chlorcarbonyl, substituiert ist.

Die Kondensation einer freien Carbonsäure mit dem entsprechenden Amin kann in besonders bevorzugter Weise weise in Gegenwart eines der folgenden Kondensationsmittel durchgeführt werden: N,N'-disubstituierte Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise N,N'-Carbonylimidazol, 1,2-oxazoliumverbindungen, z. B. 2-Ethyl-5-phenyl-1,2-oxazolium-3' -sulfonat und 2-tert-Butyl-5-methylisoxazoliumperchlorat, oder eine geeignete Acylaminoverbindung, z. B. 2-Ethoxy-1-ethoxycarbonyl-1,2-dihydrochinolin, N,N,N',N'-Tetraalkyluroniumverbindungen, wie O-Benztriazol-1-yl-N,N,N',N'-tetra-methyluronium-hexafluorphosphat, ferner aktivierte Phosphorsäurederivate, z. B. Diphenylphosphorylazid, Diethylphosphorylcyanid, Phenyl-N-phenylphosphoroamidochloridat, Bis-(2-oxo-3-oxazolidinyl)phosphinsäurechlorid oder 1-Benztriazolyloxy-tris-(dimethylamino)-phosphonium-hexafluorophosphat, wobei gegebenenfalls racemisierungshemmende Stoffe zugesetzt werden, wie 1-Hydroxybenztriazol.

Die Kondensation aktivierter Ester, reaktionsfähiger Anhydride oder reaktionsfähiger cyclischer Amide mit den entsprechenden Aminen, auch, wenn diese nur in situ gebildet werden, wird üblicherweise in Gegenwart einer organischen Base, wie einfachen Triniederalkylaminen, z. B. Triethylamin oder Tributylamin, oder Triniederalkylamins mit voluminösen Resten, z. B. Ethyldiisopropylamin, und/oder einer heterocyclischen Base, z. B. Pyridin, 4-Dimethylaminopyridin oder bevorzugt n-Methylmorpholin, durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z. B. in Gegenwart von anorganischen Carbonaten, z. B. Ammonium- oder Alkalimetallcarbonaten oder -hydrogencarbonaten, wie Natrium- oder Kaliumcarbonat oder -hydrogencarbonat (üblicherweise zusammen mit einem Sulfat), erfolgen.

Carbonsäurechloride, beispielsweise die von der Säure der Formel V abgeleiteten Chlorkohlensäurederivate, werden mit den entsprechenden Aminen vorzugsweise in Gegenwart eines organischen Amins, z. B. der vorstehend genannten Triniederalkylamine oder heterocyclischen Basen, gegebenenfalls in Gegenwart eines Hydrogensulfates, kondensiert.

In analoger Weise lassen sich viele der oben für Carbonsäuren der Formeln V aufgeführten Reaktionstypen auch für Verbindungen der Formel V mit endständiger Sulfonylgruppe (Niederalkansulfonsäuren) bei der Kondensation mit Verbindungen der Formel V zu Sulfonamiden durchführen.

So können beispielsweise aktivierte Sulfonsäureester eingesetzt werden, z. B. die entsprechenden, insbesondere durch Nitrogruppen substituierten, Arylester, wie Phenylester, wobei die Aminkomponente der Formel IV auch als Alkalimetallamid, z. B. Alkalimetallarylamid, wie Natriumanilinamid, oder Alkalimetallsalz von stickstoffhaltigen Heterocyclen, z. B. Kalium-pyrrolid, eingesetzt werden kann.

Ferner können reaktionsfähige Anhydride zum Einsatz kommen, wie etwa die entsprechenden symmetrischen (herstellbar z. B. durch Reaktion der alkansulfonsauren Silbersalze mit Alkansulfonylchloriden) oder vorzugsweise asymmetrischen Säureanhydride, z. B. Anhydride mit anorganischen Säuren, wie Sulfonylhalogenide, insbesondere Sulfonylchloride (erhältlich z. B. durch Umsetzung der entsprechenden Sulfonsäuren mit anorganischen Säurechloriden, z. B. Thionylchlorid, Phosphorpentachlorid), mit organischen Carbonsäuren (erhältlich z. B. durch Behandeln eines Sulfonsäurehalogenides mit dem Salz einer Carbonsäure, wie einem Alkalimetallsalz, analog der oben genannten Methode der gemischten Sulfonsäureanhydride), oder Azide (erhältlich z. B. aus einem entsprechenden Sulfonsäurechlorid und Natriumazid oder über das entsprechende Hydrazid und dessen Behandlung mit salpetriger Säure analog der oben genannten Azidmethode).

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z. B. Formamid oder Dimethylformamid, einem halogenierten Kohlenwasserstoff, z.B. Methylenchlorid, Tetrachlorkohlenstoff oder Chlorbenzol, einem Keton, z. B. Aceton, einem cyclischen Ether, z. B. Tetrahydrofuran, einem Ester, z. B. Essigsäureethylester, oder einem Nitril, z. B. Acetonitril, oder in einer Mischung davon, gegebenenfalls bei erniedrigter oder erhöhter Temperatur, z. B. in einem Temperaturbereich von etwa -40 °C bis etwa + 100 °C, bevorzugt von etwa - 10 °C bis etwa +50 °C, und ohne Inertgas oder unter Inertgas-, z. B. Stickstoff- oder Argonatmosphäre.

Auch wässrige, beispielsweise alkoholische, z. B. Ethanol, oder aromatische Lösungsmittel, z. B. Benzol oder Toluol, sind möglich. Bei Gegenwart von Alkalihydroxiden als Basen kann gegebenenfalls auch Aceton zugesetzt werden.

Die Kondensation kann auch gemäss der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc Natl. Acad. Sci. USA 82, 5131 - 5135 (1985) beschrieben ist.

Die Freisetzung durch Schutzgruppen geschützter funktioneller Gruppen in den erhaltenen Verbindungen der Formel I mit geschützten Funktionen erfolgt nach einer oder mehreren der unter Verfahren a) genannten Methoden.

Verfahren c) (Kondensation unter Bildung einer Amidbindung)

In Ausgangsmaterialien der Formeln VI und VII sind funktionelle Gruppen mit Ausnahme der Gruppen, die an der Reaktion teilnehmen sollen oder unter den Reaktionsbedingungen nicht oder nur in geringem Masse reagieren, unabhängig voneinander durch Schutzgruppen geschützt.

Die Kondensation zur Bildung der Amidbindung unter Erhalt einer Verbindung der Formel I, in der einzelne oder mehrere funktionelle Gruppen gegebenenfalls geschützt vorliegen, erfolgt auf analoge Weise, wie unter Verfahren b) beschrieben. Auch die entsprechenden reaktionsfähigen Säurederivate und reaktionsfähigen Aminoderivate sind analog den für Verfahren b) beschriebenen.

Die Freisetzung durch Schutzgruppen geschützter funktioneller Gruppen in den erhaltenen Verbindungen der Formel I mit geschützten Funktionen erfolgt nach einer oder mehreren der unter Verfahren a) genannten Methoden.

Verfahren d) (Kondensation unter Bildung einer Amidbindung)

In Ausgangsmaterialien der Formeln VIII und IX sind funktionelle Gruppen mit Ausnahme der Gruppen, die an der Reaktion teilnehmen sollen oder unter den Reaktionsbedingungen nicht oder nur in geringem Masse reagieren, unabhängig voneinander durch Schutzgruppen geschützt.

Die Kondensation zur Bildung der Amidbindung unter Erhalt einer Verbindung der Formel I, in der einzelne oder mehrere funktionelle Gruppen gegebenenfalls geschützt vorliegen, erfolgt auf analoge Weise, wie unter Verfahren b) beschrieben. Auch die entsprechenden reaktionsfähigen Säurederivate und reaktionsfähigen Aminoderivate sind analog den für Verfahren b) beschriebenen.

Die Freisetzung durch Schutzgruppen geschützter funktioneller Gruppen in den erhaltenen Verbindungen der Formel I mit geschützten Funktionen erfolgt nach einer oder mehreren der unter Verfahren a) genannten Methoden.

Verfahren e) (Kondensation unter Bildung einer Amidbindung)

In Ausgangsmaterialien der Formeln X und XI sind funktionelle Gruppen mit Ausnahme der Gruppen, die an der Reaktion teilnehmen sollen oder unter den Reaktionsbedingungen nicht oder nur in geringem Masse reagieren, unabhängig voneinander durch Schutzgruppen geschützt.

Die Kondensation zur Bildung der Amidbindung unter Erhalt einer Verbindung der Formel I, in der einzelne oder mehrere funktionelle Gruppen gegebenenfalls geschützt vorliegen, erfolgt auf analoge Weise, wie unter Verfahren b) beschrieben. Auch die entsprechenden reaktionsfähigen Säurederivate sind analog den für Verfahren b) beschriebenen, die reaktionsfähigen Iminoderivate der Formel IX sind analog den reaktionsfähigen unter Verfahren b) beschriebenen Aminoderivaten, soweit dies chemisch möglich ist.

Die Freisetzung durch Schutzgruppen geschützter funktioneller Gruppen in den erhaltenen Verbindungen der Formel I mit geschützten Funktionen erfolgt nach einer oder mehreren der unter Verfahren a) genannten Methoden.

Zusätzliche Verfahrensmassnahmen

Bei den zusätzlichen Verfahrensmassnahmen, die gewünschtenfalls durchgeführt werden, liegen funktionelle Gruppen der Ausgangsverbindungen, die nicht an der Reaktion teilnehmen sollen, ungeschützt oder erforderlichenfalls in geschützter Form, beispielsweise durch eine oder mehrere der oben unter Verfahren a) genannten Schutzgruppen, vor. Die Schutzgruppen werden auf einmal oder schrittweise nach einer oder mehreren der unter Verfahren a) genannten Methoden abgespalten. Hierbei sei ausdrücklich darauf verwiesen, dass eine Aminogruppe als Azidogruppe geschützt vorliegen kann und dass die Freisetzung der Aminogruppe aus der Azidogruppe im Rahmen des vorliegenden Textes auch als Schutzgruppenabspaltung betrachtet wird.

Salze von Verbindungen der Formel I mit mindestens einer salzbildenden Gruppe können in an sich bekannter Weise hergestellt werden. So kann man Salze von Verbindungen der Formel I mit sauren Gruppen z. B. durch Behandeln mit Metallverbindungen, wie Alkalimetallsalzen von geeigneten organischen Carbonsäuren, z. B. dem Natriumsalz der 2-Ethyl-hexansäure, mit organischen Alkali- oder Erdalkalimetallverbindungen, wie den entsprechenden Hydroxiden, Carbonaten oder Hydrogencarbonaten, wie Natrium- und Kaliumhydroxid, -carbonat oder -hydrogencarbonat, mit entsprechenden Calciumverbindungen oder mit Ammoniak oder einem geeigneten organischen Amin bilden, wobei man vorzugsweise stöchiometrische Mengen oder nur einen kleinen Überschuss des salzbildenden Mittels verwendet. Säureadditionssalze von Verbindungen der Formel I erhält man in üblicher Weise, z. B. durch Behandeln mit einer Säure oder einem geeigneten Anionenaustauscherreagenz. Innere Salze von Verbindungen der Formel I, welche saure und basische salzbildende Gruppen enthalten, z. B. eine freie Carboxygruppe und eine freie Aminogruppe, können z.B. durch Neutralisieren von Salzen, wie Säureadditionssalzen, auf den isoelektrischen Punkt, z. B. mit schwachen Basen, oder durch Behandeln mit Ionenaustauschern gebildet werden.

Salze können in üblicher Weise in die freien Verbindungen überführt werden, Metall- und Ammoniumsalze beispielsweise durch Behandeln mit geeigneten Säuren oder sauren Ionenaustauschern, und Säureadditionssalze beispielsweise durch Behandeln mit einem geeigneten basischen Mittel oder basischen Ionenaustauschern.

Die Umwandlung eines Salzes einer Verbindung der Formel I in ein anderes kann über die freie Verbindung oder durch direkten Austausch des salzbildenden Ions erfolgen, beispielsweise durch Ionenaustauschchromatographie.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z. B. optisch aktiven Säuren oder Basen, durch Chromatographie an mit optisch aktiven Verbindungen belegten Säulenmaterialien oder durch enzymatische Methoden, z. B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

In einer erhältlichen Verbindung der Formel I kann man eine Amino- oder Carboxamidgruppe substituieren, eine in freier oder in reaktionsfähiger Form vorliegende Carboxygruppe verestern oder amidieren bzw. eine veresterte oder amidierte Carboxygruppe in eine freie Carboxygruppe überführen.

Die Substitution einer Aminogruppe durch Alkylierung unter Einführung von Niederalkyl $R_1$ zur Herstellung von Verbindungen der Formel I, in denen $R_2$ einen der oben genannten Reste ausser Wasserstoff oder Niederalkyl bedeutet, und/oder zur Alkylierung von Verbindungen der Formel I, in denen $R_1$ Piperazinocarbonyl bedeutet, zur Herstellung der entsprechenden N-$C_1$-$C_4$-Alkyl-piperazinoverbindungen erfolgt z. B. durch Alkylierung.

Geeignete Mittel zur Alkylierung einer Verbindung der Formel I sind beispielsweise Diazoverbindungen, z. B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z. B. in Gegenwart eines Edelmetalls in fein verteilter Form, z. B. Kupfer, oder eines Edelmetallsalzes, z. B. Kupfer(I)-chlorid oder Kupfer(II)-sulfat.

Alkylierungsmittel sind auch in der Deutschen Offenlegungsschrift 2 331 133 genannt, welche man unter den dort genannten Reaktionsbedingungen mit einer Verbindung der Formel I mit einer Carboxamidgruppe umsetzen kann.

Weitere Alkylierungsmittel sind ausgewählt aus entsprechenden Niederalkylverbindungen, die einen Substituenten X tragen, worin X eine Abgangsgruppe ist. Eine Abgangsgruppe ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff-

oder Iodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure, verestertes Hydroxy oder mit Stickstoffwasserstoffsäure verestertes Hydroxy.

Die Reaktion kann unter den Bedingungen einer nukleophilen Substitution erster oder zweiter Ordnung ablaufen, beispielsweise, wie unter Verfahren a) beschrieben.

In einer erhältlichen Verbindung der Formel I, worin die Substituenten $R_1$, $R_2$ und $R_3$ die genannten Bedeutungen haben, $R_4$ eine freie Hydroxygruppe bedeutet, und die übrigen funktionellen Gruppen in geschützter Form vorliegen, kann man die freie Hydroxygruppe mit einem $C_1$-$C_4$-Alkylrest verethern.

Die Veretherung kann mit aus den oben genannten Alkylierungsmitteln ausgewählten, zur Einführung von $C_1$-$C_4$-Alkylresten geeigneten Reagentien und unter den oben beschriebenen Reaktionsbedingungen erfolgen, z. B. mit Diazomethan, $C_1$-$C_4$-Alkylhalogeniden, Sulfonsäureestern, Meerweinsalzen, 1-substituierten 3-Aryltriazenen, etc.

In einer erhältlichen Verbindung der Formel I kann man eine Thiogruppe, z. B. in Thiomorpholinocarbonyl $R_2$ und/oder Thiomorpholino $R_3$, zu einer Sulfinyl- oder Sulfonylgruppe oder eine Sulfinylgruppe, wie in S-Oxo-Thiomorpholinocarbonyl $R_2$ und/oder S-Oxo-thiomorpholino $R_3$, zu einer Sulfonylgruppe oxidieren.

Die Oxidation zur Sulfonylgruppe kann mit den meisten der üblichen Oxidationsmittel durchgeführt werden. Vorzugsweise verwendet man solche Oxidationsmittel, die die Thiogruppe oder Sulfinylgruppe selektiv in Gegenwart anderer funktioneller Gruppen der Verbindung der Formel I, z.B. der Amidfunktion oder der Hydroxygruppe, oxidieren, beispielsweise aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffen, z.B. Methylenchlorid oder Chloroform, Ethern, wie Diethylether oder Dioxan, Estern, wie Essigsäureethylester oder dergleichen, bei Temperaturen zwischen -78°C und Raumtemperatur, z.B. zwischen -20°C und +10°C, bevorzugt um 0°C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z.B. Methanol-Wasser oder Ethanol-Wasser, oder in wässriger Essigsäure bei Temperaturen zwischen -70°C und +30°C, z.B. zwischen -20°C und Raumtemperatur, ferner Natriummetaperiodat in Methanol oder Methanol-Wasser-Gemischen bei Temperaturen zwischen 0°C und 50°C, z.B. um Raumtemperatur.

Für die Oxidation der Thiogruppe zur Sulfinylgruppe werden selektive Oxidationsmittel in äquimolaren Mengen oder nur geringem Überschuss unter kontrollierten Reaktionsbedingungen verwendet, um eine Überoxidation zur Sulfonylgruppe zu vermeiden. Geeignet sind beispielsweise Natriummetaperiodat in Methanol oder Methanol-Wasser-Gemischen bei Temperaturen zwischen - 15°C und Raumtemperatur, z.B. um 0°C, m-Chlorperbenzoesäure in Methylenchlorid, Chloroform oder Essigester bei Temperaturen zwischen -78°C und 10°C, bevorzugt zwischen -30°C und 0°C, ferner tert-Butylhypochlorit in Niederalkanolen, z.B. Methanol, oder Wasserstoffperoxid in Aceton oder Essigsäure bei Temperaturen um 0°C, oder das oben genannte Kaliumperoxomonosulfat bei tiefen Temperaturen.

In einer erhältlichen Verbindung der Formel I mit einer Sulfinylgruppe kann man diese Gruppe zu einer Thiogruppe reduzieren. Bevorzugt sind selektive Reduktionsmittel, die andere funktionelle Gruppen der Verbindung der Formel I, z.B. die Amidfunktion, unverändert lassen. Beispiele für solche selektiven Reduktionsmittel sind Dichlorboran, das vorzugsweise in Tetrahydrofuran oder Dimethoxyethan bei Temperaturen zwischen -30°C und + 10°C eingesetzt wird, Triphenylphosphin in siedendem Tetrachlorkohlenstoff, Trichlorsilan oder Hexachlordisilan,, Eisenpentacarbonyl, ferner Natriumhydrogensulfit in wässrig-alkoholischen Lösungsmitteln, z.B. Wasser-Methanol, Wasser-Ethanol oder auch Wasser-Tetrahydrofuran, bei Temperaturen zwischen -10°C und +50°C, ferner Natriumborhydrid in Gegenwart von Kobalt(II)chlorid oder auch Wasserstoff in Gegenwart von katalytischen Mengen Palladium, z.B. Palladium/Kohle in siedendem Ethanol.

Gewünschtenfalls kann in einer erhältlichen Verbindung der Formel I eine Sulfonylgruppe zu einer Thiogruppe reduziert werden, beispielsweise mit Diisobutylaluminiumhydrid in Ether oder Tetrahydrofuran.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemässen Verfahren erhältliche Verbindung unter den Verfahrensbedingungen erzeugt und in situ weiterverarbeitet. Dabei geht man vorzugsweise von solchen Ausgangsstoffen aus, die zu den Verbindungen führen, die oben als bevorzugt beschrieben sind.

Ausgangsverbindungen:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Bei der Herstellung aller Ausgangsmaterialien können freie funktionelle Gruppen, die nicht an der jeweiligen Reaktion teilnehmen sollen, ungeschützt oder in geschützter Form vorliegen, vorzugsweise geschützt, insbesondere durch die oben unter Verfahren a) genannten Schutzgruppen. Diese Schutzgruppen können zu geeigneten Zeitpunkten durch die unter Verfahren a) beschriebenen Reaktionen freigesetzt werden. Die Verbindungen mit salzbildenden Gruppen können jeweils auch als Salze Verwendung finden, sofern die Reaktionen dies zulassen, und Salze auf jeder Stufe hergestellt oder wieder in die freien Verbindungen überführt werden.

In den Formeln können, sofern nicht die Stereochemie asymmetrischer Kohlenstoffatome durch die Wahl entsprechender Bindungssymbole definiert wird, die asymmetrischen Kohlenstoffatome in der (S)-, (R)- oder (R,S)-Konfiguration vorliegen.

Die Aminoverbindungen der Formel II können nach an sich bekannten Verfahren hergestellt werden.

Beispielsweise können sie aus Verbindungen der Formel I hergestellt werden, in denen $R_1$ Wasserstoff bedeutet, $R_2$ eine unter den Bedingungen der unter Verfahren a) genannten Schutzgruppenabspaltungsverfahren abspaltbare Gruppe bedeutet, z. B. Niederalkoxycarbonyl, Phenyl- oder Naphthylniederalkoxycarbonyl, und die übrigen Reste die genannten Bedeutungen haben, indem man die entsprechenden Reste $R_2$ abspaltet. Die entsprechenden Verbindungen der Formel I lassen sich beispielsweise herstellen nach einem der unter Verfahren c), d) oder e) genannten Verfahren, deren Ausgangsverbindungen wie unten beschrieben hergestellt werden können.

Die Verbindungen der Formel III sind bekannt oder können nach an sich bekannten Verfahren hergestellt werden, oder sie sind kommerziell erhältlich.

Die Aminoverbindungen der Formel IV können nach an sich bekannten Verfahren hergestellt werden.

Beispielsweise können sie aus Verbindungen der Formel I hergestellt werden, in denen $R_2$ eine unter den Bedingungen der unter Verfahren a) genannten Schutzgruppenabspaltungsverfahren abspaltbare Gruppe bedeutet, z. B. Niederalkoxycarbonyl, Phenyl- oder Naphthylniederalkoxycarbonyl, und die übrigen Reste die genannten Bedeutungen haben, indem man die entsprechenden Reste $R_2$ abspaltet. Die hierzu notwendigen Verbindungen der Formel I lassen sich beispielsweise herstellen nach einem der unter Verfahren a), c), d) oder e) genannten Verfahren, wobei im Falle von Verfahren a) eine den genannten abspaltbaren Resten $R_2$ entsprechende Schutzgruppe in der geschützten Ausgangsverbindung der Formel II vorhanden ist, und in den übrigen Verfahren $R_2$ ebenfalls eine entsprechende Bedeutung hat.

Die Säuren der Formel V sind bekannt oder können nach an sich bekannten Verfahren hergestellt werden, oder sie sind kommerziell erhältlich.

Aminoverbindungen der Formel VI sind bekannt oder nach an sich bekannten Verfahren herstellbar, beispielsweise durch Peptidsynthese aus (D)-, (L)- oder (D,L)-Valin, (D)-, (L)-oder (D,L)-($R_4$-Phenyl)alanin und einer Verbindung der Formel X oder entsprechenden geschützten Derivaten unter Kondensationsbedingungen analog den unter Verfahren b) genannten.

Vorzugsweise werden sie wie folgt hergestellt:

Eine Verbindung der Formel XII,

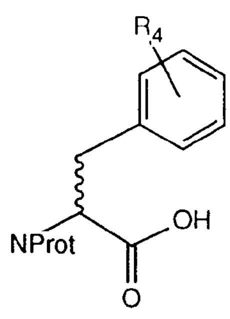

(XII)

worin NProt eine geschützte Aminogruppe, vorzugsweise Arylniederalkoxycarbonylamino, wie unter Verfahren a) beschrieben, z. B. Phenylniederalkoxycarbonylamino, wie Benzyloxycarbonylamino, bedeutet und $R_4$ die für Verbindungen der Formel 1 genannten Bedeutungen hat, insbesondere die entsprechende Verbindung, die am asymmetrischen C-Atom die (L)-Konfiguration hat, oder ein entsprechendes Säurederivat hiervon, z.

B. analog zu den unter Verfahren b) beschriebenen Säurederivaten, wird unter Reaktionsbedingungen analog zu den unter Verfahren b) genannten mit einer Verbindung der Formel X kondensiert, vorzugsweise in einem inerten Lösungsmittel, wie einem chlorierten Kohlenwasserstoff, z. B. Methylenchlorid, bei Temperaturen zwischen -80 und 50 °C, insbesondere zwischen -10 und 30 °C, in Gegenwart eines Kondensationsmittels, z. B. eines geeigneten N,N'-disubstituierten Carbodiimids, z. B. N,N'-Dicyclohexylcarbodiimid (Carbodiimid-Methode).

Man erhält eine Verbindung der Formel XIII,

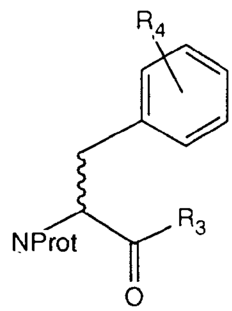

(XIII),

worin $R_3$ die für Verbindungen der Formel I genannten Bedeutungen hat und die übrigen Reste die genannten Bedeutungen haben. Die Freisetzung der Schutzgruppe an der geschützten Aminogruppe NProt erfolgt analog den unter Verfahren a) genannten Bedingungen, vorzugsweise im Falle der die Abspaltung von Arylniederalkoxycarbonyl durch katalytische Hydrierung, insbesondere in Gegenwart von Edelmetall-Katalysatoren, wie Platin, Rhodium oder Palladium, gegebenenfalls in Gegenwart von Trägermaterialien, wie Silikaten, Aktivkohle oder Aluminiumoxiden, z. B. mit Palladium auf Aktivkohle, in polaren Lösungsmitteln, z. B. Alkoholen, wie Methanol oder Ethanol, bei Temperaturen von -80 bis 50 °C, insbesondere von -10 bis 30 °C. Man erhält eine Verbindung der Formel VIII,

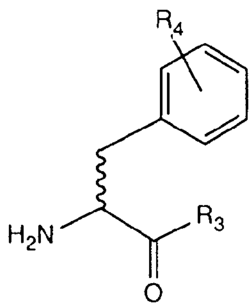

(VIII)

worin die Reste die genannten Bedeutungen haben. Zur Einführung des Restes (L)-, (D)-oder (D,L)-Valin wird die Aminoverbindung der Formel VIII mit einer Carbonsäure der Formel XIV,

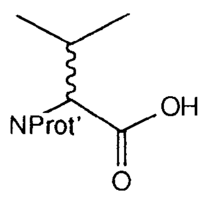

(XIV)

oder einem rekationsfähigen Säurederivat hiervon, worin NProt' eine geschützte Aminogruppe, wie oben für NProt beschrieben, bedeutet, unter Bedingungen analog den für Verfahren b) beschriebenen kondensiert, vorzugsweise unter den für die Herstellung der Formel XIII genannten Bedingungen, und die Schutzgruppe wie unter Verfahren a) beschrieben abspaltet, vorzugsweise unter den Bedingungen, wie oben bei der Freisetzung der Schutzgruppen aus Verbindungen der Formel XIII beschrieben. Man erhält eine Verbindung der Formel

VI, in der die Reste die genannten Bedeutungen haben.

Verbindungen der Formel VII lassen sich nach an sich bekannten Verfahren herstellen.

Vorzugsweise geht man von dem Lacton der Formel XV aus,

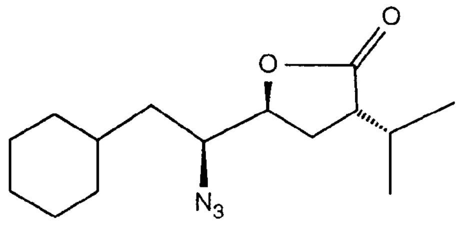

(XV)

welcher hergestellt wird gemäss dem in J. Org. Chem. 54, 1178 - 1186 (1989) beschriebenen Verfahren. Diese Verbindung wird unter Lactonspaltung hydrolysiert, vorzugsweise in Gegenwart von alkalischen Verbindungen, insbesondere Hydroxiden, z. B. Alkalimetallhydroxiden, wie Natriumhydroxid oder Kaliumhydroxid, in wässrigen Lösungsmitteln, z.B. Alkohol/wasser-Gemischen, wie Methanol/Wasser oder Ethanol/Wasser, bei Temperaturen zwischen -80 und 50 °C, insbesondere zwischen -10 und 30 °C, unter Erhalt der entsprechenden freien Carbonsäure (5(S)-Azido-4(S)-hydroxy-6-cyclohexyl-2(S)-isopropylhexansäure) oder des entsprechenden Salzes, z. B. des Alkalimetallsalzes, wie des Natriumsalzes. Hieraus werden nun die Verbindungen der Formel VII folgendermassen hergestellt:

Zunächst werden die freie oder als Salz vorliegende Carboxygruppe und die freie Hydroxygruppe geschützt unter Einführung einer der unter Verfahren a) beschriebenen Schutzgruppen. Vorzugsweise wird an beiden Gruppen eine Triniederalkylsilyl-Gruppe eingeführt, wie tert-Butyl-dimethylsilyl, insbesondere durch Umsetzung von 5(S)-Azido-4 (S)-hydroxy-6-cyclohexyl-2(S)-isopropylhexansäure oder des entsprechenden Salzes, z. B. des Alkalimetallsalzes, wie des Natriumsalzes, mit dem entsprechenden Triniederalkylhalogensilan, z. B. Triniederalkylchlorsilan, wie tert-Butyl-dimethylchlorsilan, in Ab- oder vorzugsweise Anwesenheit von Basen, wie cyclischen oder acyclischen Stickstoffbasen, z. B. Imidazol, in einem aprotischen Lösungsmittel, insbesondere einem Carbonsäureamid, wie Dimethylformamid, bei Temperaturen zwischen -80 und 50 °C, insbesondere zwischen -10 und 30 °C, und in Ab- oder vorzugsweise Anwesenheit eines Schutzgases, wie Stickstoff oder Argon.

Verbindungen der Formel VII, worin $R_1$ und/oder $R_2$ Niederalkyl oder Wasserstoff bedeutet, wobei wenigstens einer der beiden Reste nicht Wasserstoff bedeutet, lassen sich hieraus herstellen, indem man die so erhaltene Verbindung der Formel XVI,

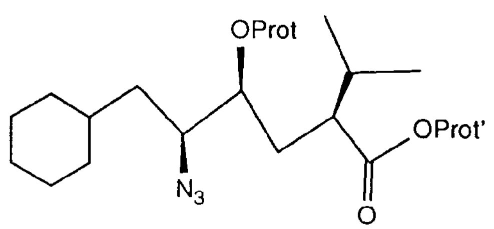

(XVI)

worin OProt und OProt' verschiedene oder vorzugsweise die gleiche derjenigen der genannten Schutzgruppen bedeutet, welche nicht durch Hydrierung abgespalten werden, insbesondere einen der genannten Triniederalkylsilylreste, unter Reduktion des Azidrestes hydriert, vorzugsweise in Gegenwart eines Katalysators, z. B. eines Edelmetallkatalysators, wie Platin, Rhodium oder Palladium, welcher frei oder an einen Träger gebunden vorliegt, z. B. an Silicagel, Aluminiumoxid oder Aktivkohle, z. B. Palladium an Aktivkohle, in einem polaren Lösungsmittel, wie einem Alkohol, z. B. Methanol oder Ethanol, bei Temperaturen zwischen -80 und 50 °C, insbesondere zwischen -10 und 30 °C, unter Normaldruck oder bei bis zu 5 atm Wasserstoffdruck.

Man erhält hierbei eine Verbindung der Formel XVII,

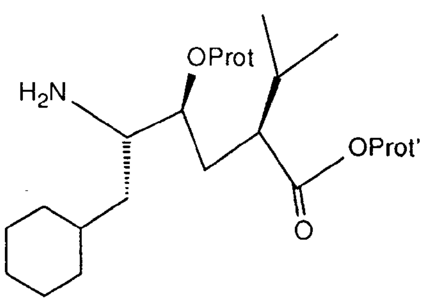

(XVII)

worin die Reste die zuletzt genannten Bedeutungen haben. Hieraus kann direkt durch Abspaltung der Schutzgruppen OProt und OProt', wie unter Verfahren a) beschrieben, wobei als organisches Silyloxycarbonyl, wie Triniederalkylsilyloxycarbonyl, z. B. Tri-methylsilyloxycarbonyl, geschütztes Carboxy in üblicher Weise solvolytisch, z. B. durch Behandeln mit Wasser, einem Alkohol, einer Base, insbesondere einem Carbonat, z. B. einem Alkalimetallcarbonat, wie Kaliumcarbonat, oder Säure, oder ausserdem Fluorid, wie unter Verfahren a) beschrieben, freigesetzt wird, vorzugsweise mit einem Alkalicarbonat in einer wässrig-alkoholischen Lösung, z. B. Methanol oder Ethanol in Wasser, bei Temperaturen zwischen -80 und 50 °C, insbesondere zwischen -10 und 30 °C, unter Schutzgas, wie Argon oder Stickstoff, während als organisches Silyloxy geschütztes Hydroxy durch Hydrolyse in Gegenwart einer Hydroxid-haltigen Base, wie eines Erdalkalihydroxides oder vorzugsweise eines Alkalimetallhydroxides, z. B. NaOH, KOH oder LiOH, oder durch Acidolyse, z. B. durch Mineralsäure, bevorzugt Fluorwasserstoffsäure, oder eine starke Carbonsäure, oder vorzugsweise ein Fluorsalz eines Amines, insbesondere tert-Butylammoniumfluorid in einem aprotischen polaren Lösungsmittel, z. B. einem Säureamid, wie Dimethylformamid, bei Temperaturen zwischen -80 und 50 °C, insbesondere zwischen -10 und 30 °C, eine Verbindung der Formel VII freigesetzt werden. Hierbei wird eine Verbindung der Formel VII erhalten, in der $R_1$ und $R_2$ Wasserstoff bedeuten. Alternativ können die Reste $R_1$ und/oder $R_2$ ausser Wasserstoff durch Alkylierung mit einer Verbindung der Formel III, wie unter Verfahren a) beschrieben, und/oder durch Acylierung mit einer Säure der Formel V, wie unter Verfahren b) beschrieben, eingeführt werden und erst danach die Schutzgruppen abgespalten werden, wobei Verbindungen der Formel VII erhalten werden, in denen maximal einer der Reste $R_1$ oder $R_2$ Wasserstoff bedeuten kann.

Verbindungen der Formel VIII können beispielsweise aus Verbindungen der Formel XIII erhalten werden, wie oben beschrieben.

Verbindungen der Formel IX können aus Carbonsäuren der Formel VII oder deren reaktionsfähigen Derivaten, in denen solche funktionellen Gruppen, die nicht an der Reaktion teilnehmen sollen, wie unter Verfahren a) beschrieben geschützt sind, durch Kondensation mit (D)-, (L)- oder (D,L)-Valin, worin die Carboxygruppe wie unter Verfahren a) beschrieben geschützt ist, unter Bildung einer Amidbindung unter den für Verfahren b) genannten Reaktionsbedingungen erhalten werden, vorzugsweise unter analogen Reaktionsbedingungen, wie bei der Umsetzung von Verbindungen der Formel VIII zu Verbindungen der Formel XIV beschrieben. Falls erforderlich, werden vorhandene Schutzgruppen wie unter Verfahren a) beschrieben abgespalten.

Die Verbindungen der Formel X sind bekannt oder nach an sich bekannten Verfahren herstellbar, oder sie sind kommerziell erhältlich.

Die Verbindungen der Formel XI schliesslich können aus Carbonsäuren der Formel IX oder deren reaktionsfähigen Derivaten unter Reaktionsbedingungen analog den unter Verfahren b), insbesondere den für die Herstellung von Verbindungen der Formel XI aus Verbindungen der Formel VII, genannten Bedingungen hergestellt werden, wobei freie funktionelle Gruppen mit Ausnahme der an der Reaktion beteiligten gegebenenfalls in geschützter Form vorliegen, und, falls erforderlich, vorhandene Schutzgruppen abgespalten werden, wie unter Verfahren a) beschrieben.

Eine bevorzugte Variante zur Herstellung von Verbindungen der Formel I geht von den wie oben beschrieben aus Verbindungen der Formel XIII und XIV hergestellten Verbindungen der Formel VI und den wie oben hergestellten Verbindungen der Formel XVI aus, in denen freie funktionelle Gruppen mit Ausnahme der an der Reaktion beteiligten gegebenenfalls in geschützter Form vorliegen, wie unter Verfahren a) beschrieben. Hierbei wird die Carboxyschutzgruppe OProt' aus einer Verbindung der Formel XVI selektiv abgespalten, vorzugsweise durch Hydrolyse in Gegenwart einer Base, wie eines Salzes der Kohlensäure, z. B. eines Alkalimetallcarbonates, wie Natriumcarbonat, in einer wässrig-alkoholischen Lösung, z. B. einem Methanol- oder Ethanol-Wasser-Gemisch, bei Temperaturen zwischen -80 und 50 °C, insbesondere zwischen -10 und 30 °C, in Ab- oder insbesondere Anwesenheit von Schutzgas, wie Stickstoff oder Argon. Man erhält eine Verbindung der Formel XVIII,

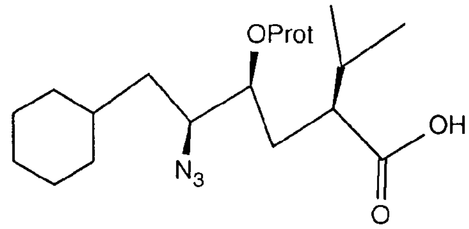

(XVIII)

worin die Reste die für Verbindungen der Formel XVI genannten Bedeutungen haben. Diese wird dann mit der Verbindung der Formel VI kondensiert unter den Bedingungen für die Herstellung einer Amidbindung, wie unter Verfahren b) beschrieben, insbesondere durch in-situ-Reaktion in Gegenwart eines Kondensationsmittels, wie Benzotriazol-1-yl-oxy -tris-(di-methylamino)-phosphonium-hexafluor-phosphat, eines sterisch gehinderten Amins, wie N-Methylmorpholin, und einer racemisierungshindernden Verbindung, wie 1-Hydroxy-benz-triazol, in einem polaren Lösungsmittel, vorzugsweise einem Säureamid, z. B. einem Diniederalkylaminoniederalkanoylamid, wie Dimethylformamid, bei Temperaturen zwischen -50 und 80 °C, insbesondere zwischen 0 und 30 °C. Aus der erhaltenen Verbindung wird die Schutzgruppe OProt abgespalten, wie für die Abspaltung von Hydroxyschutzgruppen unter Verfahren a) beschrieben, insbesondere durch Hydrolyse in Gegenwart einer Hydroxid-haltigen Base, wie eines Erdalkalihydroxides oder insbesondere eines Alkalimetallhydroxides, z. B. NaOH, KOH oder LiOH, oder durch Acidolyse, z. B. durch Mineralsäure, bevorzugt Fluorwasserstoffsäure, oder eine starke Carbonsäure, oder vorzugsweise ein Fluorsalz eines Amines, in besonders bevorzugter Weise durch tert-Butylammoniumfluorid in einem aprotischen polaren Lösungsmittel, z. B. einem Säureamid, wie Dimethylformamid, bei Temperaturen zwischen -80 und 50 °C, insbesondere zwischen -10 und 30 °C. Man erhält eine Verbindung der Formel XIX,

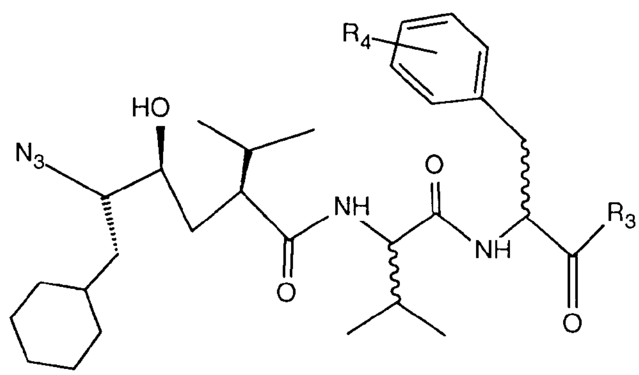

(XIX)

worin die Reste die für Verbindungen der Formel I genannten Bedeutungen haben. Hieraus erhält man eine Verbindung der Formel I, worin $R_1$ und $R_2$ Wasserstoff bedeuten, indem man die Azidgruppe reduziert, vorzugsweise hydrogeniert, insbesondere in Gegenwart eines Katalysators, z. B. eines Edelmetallkatalysators, wie Platin, Rhodium oder Palladium, welcher frei oder an einen Träger gebunden vorliegt, z. B. an Silicagel, Aluminiumoxid oder Aktivkohle, z. B. Palladium an Aktivkohle, in einem polaren Lösungsmittel, wie einem Alkohol, z. B. Methanol oder Ethanol, bei Temperaturen zwischen -80 und 50 °C, insbesondere zwischen - 10 und 30 °C, unter Normaldruck oder bei bis zu 5 atm Wasserstoffdruck.

Aus der genannten Verbindung der Formel I, in der $R_1$ und $R_2$ Wasserstoff bedeuten, werden dann gemäss einem der oben beschriebenen Verfahren a) oder b), vorzugsweise gemäss Verfahren b), die jeweils erhältlichen Verbindungen der Formel I hergestellt.

Generelle Anmerkungen zu den Verfahren:

Alle oben angeführten Verfahrensschritte werden unter an sich bekannten, vorzugsweise den spezifisch genannten, Reaktionsbedingungen durchgeführt, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, z. B. Ionenaustauschern, wie Kationenaustauschern, z. B. in der $OH^-$ oder der $H^+$-Form, je nach Art der Reaktion und/oder der Reaktionsteilnehmer bei erniedrigter, normaler oder erhöhter Temperatur, z.B. im Temperaturbereich von etwa -100°C bis etwa 120°C, vorzugsweise von etwa -80°C bis etwa 50°C, insbesondere bei - 20

bis 40 °C, z. B. bei 0 bis 25 °C, unter atmosphärischem Druck oder in einem geschlossenen Gefäss, gegebenenfalls unter Druck, und/oder in einer inerten Atmosphäre, z.B. unter einer Argon- oder Stickstoffatmosphäre.

Auf allen Reaktionsstufen können auftretende Isomerengemische in die einzelnen Isomeren, z. B. Diastereomeren oder Enantiomeren, oder in beliebige Gemische von Isomeren, z. B. Racemate oder Diastereomerengemische, aufgetrennt werden, beispielsweise analog zu den Methoden, die unter den "Zusätzlichen Verfahrensmassnahmen" beschrieben sind.

Zu den Lösungsmitteln, aus denen die für die jeweilige Reaktion geeigneten ausgewählt werden können, zählen beispielsweise Wasser, Ester, wie Niederalkylniederalkanoate, z. B. Essigsäureethylester, Ether, wie aliphatische Ether, z. B. Diethylether, oder cyclische Ether, z. B. Tetrahydrofuran, flüssige aromatische Kohlenwasserstoffe, wie Benzol oder Toluol, Alkohole, wie Methanol, Ethanol oder 1 - oder 2-Propanol, Nitrile, wie Acetonitril, Halogenkohlenwasserstoffe, wie Methylenchlorid, Chloroform oder Tetrachlormethan, Säureamide, wie Dimethylformamid, Basen, wie heterocyclische Stickstoffbasen, z. B. Pyridin, Carbonsäureanhydride, wie Niederalkansäureanhydride, z. B. Acetanhydrid, cyclische, lineare oder verzweigte Kohlenwasserstoffe, wie Cyclohexan, Hexan oder Isopentan, oder Gemische dieser Lösungsmittel, z. B. wässrige Lösungen, soweit bei der Beschreibung der Verfahren nichts anderes angegeben ist. Derartige Lösungsmittel und Lösungsmittelgemische können auch bei der Aufarbeitung, beispielsweise durch Chromatographie oder Verteilung, Verwendung finden. Bevorzugt sind die für die genannten Reaktionen jeweils spezifischen Reaktionsbedingungen.

## Pharmazeutische Präparate

Die Erfindung betrifft auch pharmazeutische Präparate, welche als Wirkstoff eine Verbindung der Formel I enthalten.

Die pharmakologisch verwendbaren Verbindungen der vorliegenden Erfindung können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, bukkalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Bevorzugt ist ein pharmazeutisches Präparat, welches geeignet ist zur Verabreichung an einen Warmblüter, insbesondere Menschen, zur Behandlung oder Prophylaxe einer auf retrovirale Aspartatproteasehemmer, wie HIV-1- oder HIV-2-Protease, ansprechenden Erkrankung, wie AIDS, welche eine wirksame, eine retrovirale Aspartatprotease, wie HIV-1- oder HIV-2-Protease, hemmende Menge einer Verbindung der Formel I oder ein Salz davon und ein oder mehrere Trägermaterialien umfasst.

Die Erfindung betrifft auch eine Methode zur Behandlung von durch Retroviren verursachten Krankheiten, z. B. von AIDS, insbesondere, wenn HIV-1 die Erkrankung verursacht, dadurch gekennzeichnet, dass eine therapeutisch gegen diese Krankheiten wirksame Menge von erfindungsgemässen Verbindungen der Formel 1, insbesondere an einen Warmblüter, vorzugsweise Menschen, der wegen einer der genannten Erkrankungen, insbesondere AIDS, einer derartigen Behandlung bedarf, verabreicht wird. Die an Warmblüter, vorzugsweise Menschen von etwa 70 kg Körpergewicht, zu verabreichenden Dosismengen liegen vorzugsweise zwischen etwa 3 mg und etwa 10 g, vorzugsweise zwischen etwa 40 mg und etwa 4 g, z. B. bei ungefähr 300 mg bis 1,5 g pro Person und Tag, verteilt auf vorzugsweise 1 bis 3 Einzeldosen, die z. B. gleich gross sein können. Üblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z. B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wässrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung

des osmotischen Druckes und/oder Puffer enthalten und werden in an sich bekannter Weise, z. B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäüre, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E,β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Miglyol 812" (Triglycerid gesättigter Fettsäuren der Kettenlänge $C_8$ bis $C_{12}$ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und, wenn erwünscht oder notwendig, nach Zugabe von geeigneten Hilfsstoffen, zu Tabletten, Dragée-Kernen oder Kapseln verarbeitet. Dabei kann man die Wirkstoffe auch in Kunststoffträger einbauen, die sie dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, beigefügt werden.

Die folgenden Beispiele dienen zur Illustration der Erfindung, schränken deren Umfang jedoch in keiner Weise ein.

Temperaturen werden in Celsiusgraden (°C) angegeben. Die $R_f$-Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt:

DC-Laufmittelsysteme

| | | |
|---|---|---|
| A: | Chloroform/Methanol/Wasser/Essigsäure | (75:27:5:0,5) |
| B: | Hexan/Essigsäureethylester | (1:1) |
| C: | Essigsäureethylester | |
| D: | Methylenchlorid/Methanol | 9:1 |

Die Abkürzung "$R_f$(A)" bedeutet beispielsweise, dass der $R_f$-Wert im Lösungsmittelsystem A ermittelt wurde. Das Mengenverhältnis von Lösungsmitteln zueinander ist stets in Volumenanteilen (v/v) angegeben. Auch bei der Definition der Fliessmittel-Systeme für die Säulenchromatographie werden die Mengenverhältnisse der verwendeten Lösungsmittel in Volumenanteilen (v/v) angegeben.

HPLC-Systeme:

Säule:

Nucleosil-C18® (Macherey & Nagel, Düren), 5μ, 250 x 4,6 mm, Durchflussrate 1 ml/min, Detektion bei 215 nm.

Laufmittel:

I: 20 % -> 100 % Acetonitril/0,1 % Trifluoressigsäure in Wasser/0,1 % Trifluoressigsäure während 35 min.

II: 0% -> 40 % Acetonitril/0,1 % Trifluoressigsäure in Wasser/0,1 % Trifluoressigsäure während 30 min.

Die weiteren verwendeten Kurzbezeichnungen und Abkürzungen haben die folgenden Bedeutungen:

| | |
|---|---|
| atm | physikalische Atmosphären (Druckeinheit) - 1 atm entspricht 1,013 bar |
| Boc | tert-Butoxycarbonyl |
| BOP | Benzotriazol-1-yl-oxy-tris-(di-methylamino)-phosponium-hexafluorphosphat |
| Cha | Cyclohexylalanin |
| DC | Dünnschichtchromatographie |
| DCC | Dicyclohexylcarbodiimid |
| DMF | Dimethylformamid |
| Essigester | Essigsäureethylester |
| Ether | Diethylether |
| FAB-MS | Fast-Atom-Bombardment-Massenspektroskopie |
| h | Stunde(n) |
| HOBt | 1-Hydroxy-benztriazol |
| min | Minute(n) |
| Pd/C | Palladium auf Aktivkohle (Katalysator) |
| RT | Raumtemperatur |
| Sole | gesättigte Kochsalzlösung |
| TBAF | Tetrabutylammoniumfluorid (Trihydrat) |
| $t_{Ret}$ | Retentionszeit |
| Z | Benzyloxycarbonyl |

Massenspektroskopische Messwerte werden nach der "Fast-Atom-Bombardment" (FAB-MS)-Methode erhalten. Die Massenangaben beziehen sich auf das protonierte Molekülion $(M+H)^+$.

Zur Bezeichnung von bivalenten Radikalen von natürlichen α-Aminosäuren werden die in der Peptidchemie üblichen Abkürzungen verwendet. Die Konfiguration am α-Kohlenstoffatom wird durch Voranstellen von (L)- oder (D)- angegeben.

Das bivalente Radikal von 5(S)-Amino-6-cyclohexyl-4(S)-hydroxy-2(S)-iso-propyl-hexansäure hat die Formel

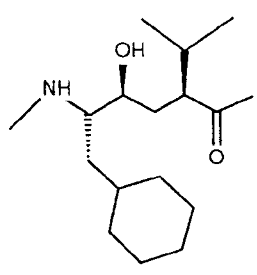

**Beispiel 1:**

**5(S)-Boc-amino-6-cyclohexyl-4(S)-hydroxy-2(S)-isopropyl-hexanoyl-(L)-valyl-(L)-phe nylalanyl-morpholin-4-ylamid**

100 mg (0,17 mMol) 5(S)-Amino-6-cyclohexyl-4(S)-hydroxy-2(S)-isopropyl-hexan oyl-(L)-valyl-(L)-phenylalanyl-morpholin-4-ylamid werden in 5 ml THF/Wasser (1:1) gelöst und bei RT mit 62 mg (0,28 mMol) Di-tert-butyl-dicarbonat versetzt. Nach 2 h wird das Lösungsmittel abgedampft, der Rückstand in Essigester gelöst und nacheinander mit gesättigter Natriumbicarbonatlösung, Wasser und Sole gewaschen. Die organischen Extrakte werden mit Wasser und Sole gewaschen, über Natriumsulfat getrocknet und eingedampft. Nach Chromatographie des Rohproduktes auf Kieselgel mit Hexan/Essigester (1:1) erhält man die Titelverbindung als amorphen Festkörper. FAB-MS:$(M+H)^+$=687, $t_{Ret}$(I)=24,7 min, DC $R_f$(A)=0,78.

Das Ausgangsmaterial wird folgendermassen hergestellt:

**1a) 5(S)-Amino-6-cylohexyl-4(S)-hydroxy-2(S)-isopropylhexanoyl-(L)-valyl-(L)phenylalanyl-(morpholin-4-yl)-amid**

Eine Lösung von 387 mg (0,63 mMol)5(S)-Azido-6-cyclohexyl-4(S)-hydroxy-2(S)-isopropyl-hexanoyl-(L)-Val-(L)-Phe-morpholin-4-ylamid in 40 ml Methanol wird in Gegenwart von 100 mg 10 % Pd/C bei RT und 1 atm Wasserstoffdruck während 3,5 h hydriert. Man filtriert vom Katalysator ab, dampft das Filtrat ein und erhält nach chromatographischer Reinigung auf Kieselgel mit Methylenchlorid/Methanol/konz. Ammoniak (90:10:0,1) die Titelverbindung als amorphen Festkörper. FAB-MS:$(M+H)^+$=587, $t_{Ret}$(I)= 14,6 min, DC $R_f$(A)=0,54.

**1b) 5(S)-Azido-6-cyclohexyl-4(S)-hydroxy-2(S)-isopropyl-hexanoyl-(L)-Val-(L)-Phemorpholin-4-ylamid**

Eine Lösung von 570 mg (0,78 mMol) 5(S)-Azido-4(S)-(tert-butyldimethylsilyloxy)-6-cyclohexyl-2(S)-isopropyl-hexanoyl-(L)-Val-(L)-Phe-morpholin-4-ylamid in 7 ml DMF wird mit 459 mg (1,57 mMol) TBAF versetzt und während 16 h bei RT gerührt. Das Reaktionsgemisch wird mit Wasser verdünnt und 3x mit Essigester extrahiert. Die organischen Extrakte werden 3x mit Wasser, 2x mit gesättigter Natriumbicarbonatlösung und 1x mit Sole gewaschen, über Natriumsulfat getrocknet und eingedampft. Das Rohprodukt wird mit Hexan digeriert und ergibt die Titelverbindung. FAB-MS: $(M+H)^+$=613, $t_{Ret}$(I)=24,7 min, DC $R_f$(B)=0,15.

**1c)5(S)-Azido-4(S)-(tert-butyldimethylsilyloxy)-6-cyclohexyl-2(S)-isopropyl-hexanoyl-(L)-Val-(L)-Phe-morpholin-4-ylamid**

Eine Lösung von 412 mg (1 mMol) 5(S)-Azido-4(S)-(tert-butyldimethylsilyloxy)-6-cyclohexyl-2(S)-isopropyl-hexansäure in 4 ml DMF (Beispiel 1h) wird mit 487 mg (1,2 mMol) BOP, 149 mg (1,1 mMol) HOBt und 330 μl (3 mMol) N-Methylmorpholin versetzt und während 30 min bei RT gerührt. Anschliessend wird eine Lösung von 400 mg (1,2 mMol) H-(L)-Val-(L)-Phe-morpholin-4-ylamid (Beispiel 1g)) in 3 ml DMF zugefügt. Nach 2,5 h wird das Reaktionsgemisch mit Wasser verdünnt und 3x mit Essigester extrahiert. Die organischen Extrakte werden mit gesättigter Natriumbicarbonatlösung, Wasser und gesättigter Natriumchloridlösung gewaschen, über Natriumsulfat getrocknet und eingedampft. Man erhält nach chromatographischer Reinigung auf Kieselgel mit einem Gradienten Hexan/Essigester (3:1 -> 1:1) die Titelverbindung als erstarrten Schaum. FAB-MS: $(M+H)^+$=727, $t_{Ret}$(I)=37,8 min, DC $R_f$(B)=0,40.

**1d) Z-(L)-Phe-morpholin-4-ylamid**

Eine Lösung von 4,49 g Z-(L)-Phe-OH in 190 ml Methylenchlorid wird auf 0 °C gekühlt und mit 3,09 g DCC versetzt. Nach 20 min Rühren bei 0 °C wird über 15 min eine Lösung von 1,31 ml Morpholin in 10 ml Methylenchlorid zugetropft. Das Reaktionsgemisch wird weitere 24 h bei RT gerührt, und nach Abfiltrieren des ausgefallenen Dicyclohexylharnstoffes wird nacheinander mit Methylenchlorid, wässriger Natriumbicarbonat-Lösung und Sole gewaschen. Nach Trocknen über Natriumsulfat und Einengen erhält man die rohe Titelverbindung, die aus Ether auskristallisiert. DC $R_f$(C)= 0,55.

**1e) H-(L)-Phe-morpholin-4-ylamid**

Eine Lösung von 5,5 g Z-(L)-Phe-morpholin-4-ylamid mit 1,5 g 10 %-igem Pd/C in 150 ml Methanol wird bei RT während 1 h mit der berechneten Menge Wasserstoff durch Hydrogenolyse in die Titelverbindung überführt. Nach Abfiltrieren des Katalysators wird eingeengt, und nach Verdünnen mit Essigester wird die erhaltene lösung mit einer gesättigten Natriumbicarbonatlösung gewaschen, über Natriumsulfat getrocknet und unter vermindertem Druck eingeengt. Nach Säulenchromatographie (analog Beispiel 1 g)) wird die Titelverbindung in reiner Form erhalten. DC $R_f$(D)= 0,3.

**1f) Z-(L)-Val-(L)-Phe-morpholin-4-ylamid**

Eine Lösung von 2,14 g Z-(L)-Val-OH in 80 ml absolutem eisgekühltem Methylenchlorid wird mit 1,75 g DCC versetzt und nach 20 min Rühren bei dieser Temperatur tropfenweise über 15 min mit einer Lösung von 2 g H-(L)-Phe-morpholin-4-ylamid versetzt. Das Reaktionsgemisch wird weitere 24 h bei RT gerührt,

und der entstandene Harnstoff wird abfiltriert. Das Filtrat wird nacheinander mit wässriger Natriumbicarbonatlösung und Sole gewaschen und nach Trocknen über Natriumsulfat eingeengt. Durch Verrühren mit Ether und Abfiltrieren des unlöslichen Rückstandes erhält man nach Einengen die Titelverbindung, die ohne zusätzliche Reinigung weiterverarbeitet wird. DC $R_f$(D)= 0,7.

**1g) H-(L)-Val-(L)-Phe-morpholin-4-ylamid**

Analog Beispiel 1 e) werden 3,9 g Z-(L)-Val-(L)-Phe-morpholin-4-ylamid durch Hydrogenolyse über 0,5 g 10 %-igem Pd/C in 150 ml Methanol in die rohe Titelverbindung überführt, die durch Säulenchromatographie ($SiO_2$, Methylenchlorid -> Methylenchlorid/ethanol: 97,5 zu 2,5 (v/v)) gereinigt wird. DC $R_f$(D)= 0,4.

**1h)5(S)-Azido-4(S)-(tert-butyldimethylsilyloxy)-6-cyclohexyl-2(S)-isopropyl-hexansäure**

Eine Suspension von 11,5 g (35,8 mMol) 5(S)-Azido-4(S)-hydroxy-6-cyclohexyl-2(S)-isopropyl-hexansäure-Natriumsalz (Beispiel 1i)) in 45 ml DMF wird nacheinander mit 11,5 g (168,9 mMol) Imidazol und 13,4 g (88,9 mMol) tert-Butyldimethylchlorsilan versetzt und während 3,75 Tagen bei RT unter Stickstoffatmosphäre gerührt. Das Reaktionsgemisch wird auf 500 ml Eiswasser gegossen und 4x mit je 250 ml eiskaltem Hexan extrahiert. Die Hexanphase wird je 2x mit Wasser und Sole gewaschen, über Natriumsulfat getrocknet und eingedampft. Nach dem Trocknen am Hochvakuum bis zur Gewichtskonstanz erhält man rohen 5(S)-Azido-4(S)-(tert-butyldimethylsilyloxy)-6-cyclohexyl-2(S)-isopropyl-hexansäure-tert-butyldimethylsilylester als hellgelbes Öl. 10,1 g (17,9 mMol) dieses Rohprodukts werden in 250 ml Methanol gelöst und bei 0°C mit einer Lösung von 2,72 g (19,7 mMol) Kaliumcarbonat in 26 ml Wasser versetzt, und die entstandene Emulsion wird während 2 h bei RT unter Argonatmosphäre gerührt. Das Reaktionsgemisch wird bei vermindertem Druck eingeeengt, mit Essigester und eiskalter, Sole verdünnt, und der pH-Wert der wässrigen Phase wird mit 0,5 M Kaliumhydrogensulfatlösung auf 2 eingestellt. Die abgetrennte wässrige Phase wird noch 3x mit Essigester extrahiert. Die vereinigten organischen Extrakte werden mit eiskaltem Wasser und eiskalter Sole gewaschen, über Natriumsulfat getrocknet und eingedampft. Das Rohprodukt besteht aus einem Gemisch von 76,5% der Titelverbindung und 23,5% des 2(R)-Epimeren. Die diastereomerenreine Titelverbindung wird nach chromatographischer Reinigung auf einer Kieselgelsäule mit einem Gradienten von Methylenchlorid -> 10% Methanol in Methylenchlorid erhalten. $t_{Ret}$(I)=37,1 min, DC $R_f$(B)=0,27.

**i)5(S)-Azido-4(S)-hydroxy-6-cyclohexyl-2(S)-isopropylhexansäure-Natriumsalz**

Eine Lösung von 1 g (3,6 mMol) (2S,4S,5S)-5-Azido-6-cyclohexyl-2-isopropyl-4 (S)-hydroxy-hexansäure-γ-lacton (=(2S,4S,5S)-5-Azido-6-cyclohexyl-2-isopropylhexanolid) (J. Org. Chem. <u>54</u>, 1178, (1989)) in 30 ml Methanol und 10 ml Wasser wird bei 0°C mit 3,6 ml (3,6 mMol) 1 N Natronlauge versetzt und während 15 h bei RT gerührt. Die Reaktionsmischung wird vollständig eingedampft und der Rückstand je 2x mit Methanol, Hexan und Methylenchlorid versetzt und wiederum vollständig eingedampft. Auf diese Weise erhält man die Titelverbindung als Rohprodukt, welches nicht weiter gereinigt wird. $t_{Ret}$(I)=22,9 min, DC $R_f$(D)=0,61.

**Beispiel 2:5(S)-Amino-6-cylohexyl-4(S)-hydroxy-2(S)-isopropylhexanoyl-(L)-valyl-(L)-phenylalanyl-(thiomorpholin-4-yl)-amid**

Ausgehend von Thiomorpholin anstelle von Morpholin, wird über Zwischenverbindungen analog Beispiel 1i) bis 1a) analog dem in Beispiel 1 beschriebenen Verfahren die Titelverbindung hergestellt.

**Beispiel 3: <u>Gelatine-Lösung</u>**

Eine sterilfiltrierte wässrige Lösung des im vorausgehenden Beispiel 1 oder 2 genannten Wirkstoffes der Formel I, die zusätzlich 20 % Cyclodextrin enthält, und eine sterile, mit Phenol konservierte Gelatinelösung werden unter Erwärmen unter aseptischen Bedingungen so vermischt, dass 1,0 ml lösung folgender Zusammensetzung resultiert:

| | |
|---|---|
| Wirkstoff | 3 mg |
| Gelatine | 150,0mg |
| Phenol | 4,7 mg |
| dest. Wasser mit 20 % Cyclodextrinen | 1,0 ml |

**Beispiel 4: Sterile Trockensubstanz zur Injektion**

Man löst 5 mg der im vorausgehenden Beispiel 1 oder 2 genannten Verbindung der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

**Beispiel 5: Nasenspray**

In einer Mischung von 3,5 ml Myglyol 812® und 0,08 g Benzylalkohol werden 500 mg fein gemahlenes (<5,0 μm) Pulver der im vorausgehenden Beispiel 1 oder 2 genannten Verbindung der Formel I als Wirkstoff suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

**Beispiel 6: Lacktabletten**

Für die Herstellung von 10 000 Tabletten enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von der im vorausgehenden Beispiel 1 oder 2 genannten Verbindung der Formel I als Wirkstoff, 50 g Maisstärke und kolloidaler Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 Min. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

**Patentansprüche**

1. Verbindungen der Formel I,

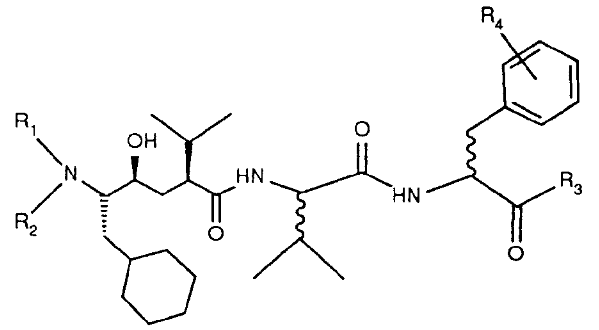

(I),

worin $R_1$ Wasserstoff oder Niederalkyl, $R_2$ Wasserstoff, Niederalkyl, Niederalkoxycarbonyl, Phenyl- oder Naphthyl-niederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu dem bindenden Stickstoffatom ein oder mehrere weitere Heteroatome ausgewählt aus unsubstituiertem oder durch einen $C_1$-$C_4$-Alkylrest substituiertem NH, O, S, S=O oder $SO_2$ als Ringglied enthält, Niederalkanoyl, Phenyl- oder Naphthylniederalkanoyl oder Niederalkansulfonyl bedeutet, $R_3$ Morpholino, Thiomorpholino, S -Oxothiomorpholino oder S,S -Dioxothiomorpholino bedeutet, wobei der Rest Heterocyclyl in Heterocyclylcarbonyl $R_2$ dieselbe Bedeutung wie $R_3$ oder eine von $R_3$ unterschiedliche Bedeutung hat, und $R_4$ Wasserstoff, Hydroxy, $C_1$-$C_4$-Alkoxy, Cyano, Trifluormethyl oder Fluor bedeuten, sowie Salze dieser Verbindungen, sofern salzbildende Gruppen vorliegen.

2. Verbindungen gemäss Anspruch 1 der Formel I, worin $R_1$ Wasserstoff bedeutet, $R_2$ Wasserstoff, Niederalkoxycarbonyl oder Heterocyclylcarbonyl bedeutet, worin Heterocyclyl 5 oder 6 Ringatome enthält, gesättigt ist und über ein Ringstickstoffatom an die Carbonylgruppe gebunden ist und zusätzlich zu dem bindenden Stickstoffatom unsubstituiertes oder durch einen $C_1$-$C_4$-Alkylrest substituiertes NH, O, S, S=O oder $SO_2$ als Ringglied enthält, $R_3$ Morpholino, Thiomorpholino, S-Oxothiomorpholino oder S,S-Dioxothiomorpholino bedeutet und $R_4$ Wasserstoff bedeutet, oder pharmazeutisch verwendbare Salze von solchen Verbindungen, die salzbildende Gruppen enthalten.

3. Verbindungen gemäss Anspruch 1 der Formel I, worin $R_1$ Wasserstoff bedeutet, $R_2$ Wasserstoff, sec- oder tert-Niederalkyl-oxycarbonyl, Piperazinocarbonyl, N-$C_1$-$C_4$-Alkyl -piperazinocarbonyl, Morpholinocarbonyl, Thiomorpholinocarbonyl, S-Oxothiomorpholinocarbonyl oder S,S-Dioxothiomorpholinocarbonyl bedeutet, $R_3$ Morpholino, Thiomorpholino, S-Oxothiomorpholino oder S,S-Dioxothiomorpholino bedeutet und $R_4$ Wasserstoff bedeutet, oder pharmazeutisch verwendbare Salze von solchen Verbindungen, die salzbildende Gruppen enthalten.

4. Verbindungen gemäss einem der Ansprüche 1, 2 oder 3 der Formel Ib

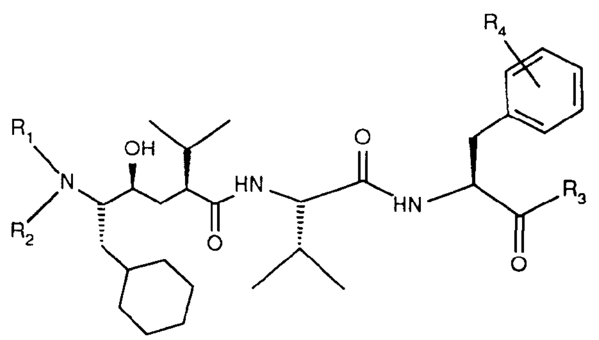

(Ib)

welche von Formel I umfasst ist, worin die Reste $R_1$, $R_2$, $R_3$ und $R_4$ die für Verbindungen der Formel I genannten Bedeutungen haben, oder pharmazeutisch verwendbare Salze von solchen Verbindungen, die salzbildende Gruppen enthalten.

5. Verbindungen gemäss Anspruch 4 der Formel Ib, worin $R_1$ Wasserstoff bedeutet, $R_2$ Wasserstoff, Niederalkoxycarbonyl oder Heterocyclylcarbonyl bedeutet, worin Heterocyclyl 5 oder 6 Ringatome enthält,

gesättigt ist und über ein Ringstickstoffatom an die Carbonylgruppe gebunden ist und zusätzlich zu dem bindenden Stickstoffatom unsubstituiertes oder durch einen $C_1$-$C_4$-Alkylrest substituiertes NH, O, S, S=O oder $SO_2$ als Ringglied enthält, $R_3$ Morpholino bedeutet, und $R_4$ Wasserstoff bedeutet, oder pharmazeutisch verwendbare Salze von solchen Verbindungen, die salzbildende Gruppen enthalten.

**6.** Verbindungen der Formel I gemäss einem der Ansprüche 1 - 5, in denen $R_3$ Morpholino bedeutet und $R_1$, $R_2$ und $R_4$ die genannten Bedeutungen haben, oder pharmazeutisch annehmbare Salze von solchen Verbindungen, die salzbildende Gruppen enthalten.

**7.** Verbindungen gemäss Anspruch 4 der Formel Ib, worin $R_1$ Wasserstoff bedeutet, $R_2$ Niederalkoxycarbonyl bedeutet, $R_3$ Morpholino bedeutet und $R_4$ Wasserstoff bedeutet.

**8.** Eine Verbindung gemäss Anspruch 4 der Formel Ib, worin $R_1$ Wasserstoff bedeutet, $R_2$ tert-Butoxycarbonyl bedeutet, $R_3$ Morpholino bedeutet und $R_4$ Wasserstoff bedeutet.

**9.** Eine Verbindung gemäss Anspruch 4 der Formel Ib, worin $R_1$ Wasserstoff bedeutet, $R_2$ tert-Butoxycarbonyl bedeutet, $R_3$ Thiomorpholino bedeutet und $R_4$ Wasserstoff bedeutet.

**10.** Verbindungen der Formel I gemäss einem der Anprüche 1 - 9, oder pharmazeutisch verwendbare Salze einer solchen Verbindung mit mindestens einer salzbildenden Gruppe, zur Anwendung in einem Verfahren zur therapeutischen Behandlung des menschlichen oder tierischen Körpers.

**11.** Pharmazeutische Präparate enthaltend Verbindungen der Formel I gemäss einem der Ansprüche 1 - 9 oder ein pharmazeutisch verwendbares Salz einer solchen Verbindung mit mindestens einer salzbildenden Gruppe zusammen mit pharmazeutisch verwendbarem Trägermaterial.

**12.** Verwendung einer der in den Ansprüchen 1 - 9 genannten Verbindungen der Formel I oder eines pharmazeutisch verwendbaren Salzes einer solchen Verbindung mit mindestens einer salzbildenden Gruppe zur Herstellung von pharmazeutischen Präparaten zur Anwendung zur Behandlung von AIDS.

**13.** Verfahren zur Herstellung einer Verbindung der Formel I gemäss Anspruch 1, worin die Substituenten die in Anspruch 1 genannten Bedeutungen haben, und deren Salzen, sofern salzbildende Gruppen vorliegen, dadurch gekennzeichnet, dass man

a) zur Herstellung von Verbindungen der Formel I, worin wenigstens einer der Reste $R_1$ oder $R_2$ Niederalkyl bedeutet und der andere Wasserstoff bedeutet oder $R_1$ und $R_2$ beide Niederalkyl bedeuten und die übrigen Reste die für Verbindungen der Formel I genannten Bedeutungen haben, eine Aminoverbindung der Formel II (welche einer Verbindung der Formel I entspricht, in der $R_1$ und $R_2$ Wasserstoff bedeuten),

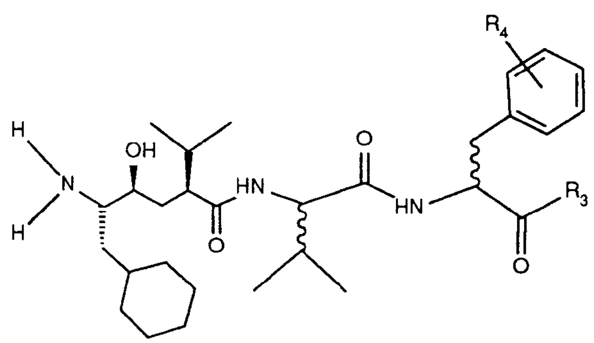

(II)

worin die $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, mit einem Alkylierungsreagenz umsetzt zur Einführung des Restes Niederalkyl $R_1$, des Restes Niederalkyl $R_2$ oder beider Reste Niederalkyl $R_1$ und Niederalkyl $R_2$ und vorhandene Schutzgruppen abspaltet, oder

b) zur Herstellung einer Verbindung der Formel I, worin $R_2$ Niederalkoxycarbonyl, Phenyl- oder Naphthyl-niederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu

dem bindenden Stickstoffatom unsubstituiertes oder durch einen $C_1$-$C_4$-Alkylrest substituiertes NH, O, S, S=O oder $SO_2$ als Ringglied enthält, Niederalkanoyl, Phenyl- oder Naphthylniederalkanoyl oder Niederalkansulfonyl bedeutet, und die übrigen Reste die genannten Bedeutungen haben, und worin freie funktionelle Gruppen mit Ausnahme der an der Reaktion beteiligten erforderlichenfalls in geschützter Form vorliegen können, eine Aminoverbindung der Formel IV,

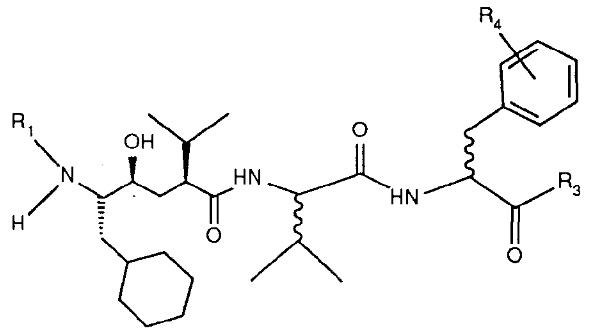

(IV)

worin die Reste $R_1$, $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, oder ein reaktionsfähiges Aminoderivat hiervon, mit einer Säure der Formel V,

$$R_2\text{-OH} \quad (V)$$

worin $R_2$ Niederalkoxycarbonyl, Phenyl- oder Naphthyl-niederalkoxycarbonyl, Heterocyclylcarbonyl, worin Heterocyclyl 5 oder 6 Ringatome enthält, gesättigt ist und über ein Ringstickstoffatom an die Carbonylgruppe gebunden ist und zusätzlich zu dem bindenden Stickstoffatom unsubstituiertes oder durch einen $C_1$-$C_4$-Alkylrest substituiertes NH, O, S, S=O oder $SO_2$ als Ringglied enthält, Niederalkanoyl, Phenyl- oder Naphthylniederalkanoyl oder Niederalkansulfonyl bedeutet, und worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet, oder

c) eine Aminoverbindung der Formel VI,

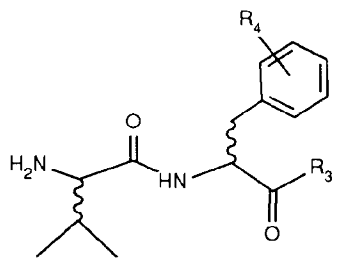

(VI)

worin $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, oder ein reaktionsfähiges Aminoderivat davon, mit einer Carbonsäure der Formel VII,

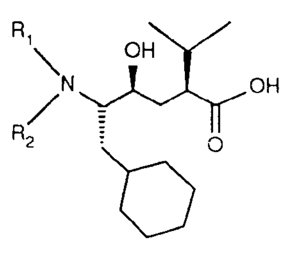

(VII)

worin die Reste $R_1$ und $R_2$ die für Verbindungen der Formel I genannten Bedeutungen haben, und worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet, oder

d) eine Aminoverbindung der Formel VIII,

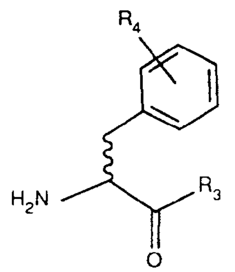

(VIII)

worin $R_3$ und $R_4$ die für Verbindungen der Formel I genannten Bedeutungen haben, und worin freie funktionelle Gruppen mit Ausnahme der an der Reaktion beteiligten erforderlichenfalls in geschützter Form vorliegen können, oder ein reaktionsfähiges Aminoderivat davon, mit einer Carbonsäure der Formel IX,

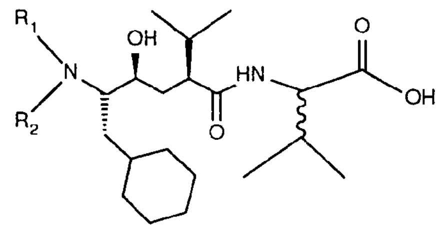

(IX)

worin $R_1$ und $R_2$ die für Verbindungen der Formel I genannten Bedeutungen haben, und worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet, oder

e) eine Verbindung der Formel X,

$R_3$-H (X)

worin $R_3$ die für Verbindungen der Formel I genannten Bedeutungen hat, und worin freie funktionelle Gruppen mit Ausnahme der an der Reaktion beteiligten erforderlichenfalls in geschützter Form vorliegen können, oder ein reaktionsfähiges Derivat davon, mit einer Carbonsäure der Formel XI

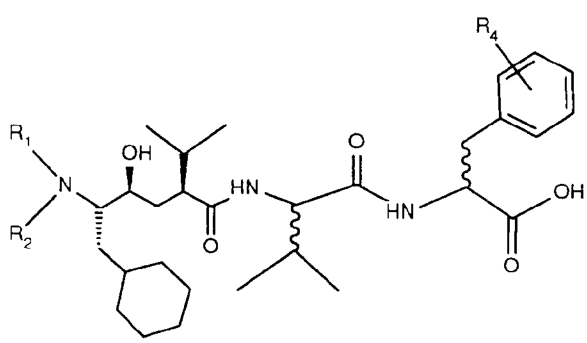

(XI)

worin $R_1$, $R_2$ und $R_4$ die für Verbindungen der Formel I genannten Bedeutungen haben, worin freie funktionelle Gruppen mit Ausnahme der an der Reaktion beteiligten erforderlichenfalls in geschützter Form vorliegen können, oder einem reaktionsfähigen Säurederivat hiervon, kondensiert, und vorhandene Schutzgruppen abspaltet,

und gewünschtenfalls eine nach einem der vorstehenden Verfahren a) bis e) erhaltene Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel I in eine andere erfindungsgemässe Verbindung der Formel I umwandelt.